# EUROPEAN PATENT APPLICATION

(11) **EP 4 647 511 A1**
(43) Date of publication of application: **12.11.2025**
(21) Application number: 24864025.2
(22) Date of filing: 21.03.2024
(51) Int. Cl.: C12Q 1/6886, C12N 15/11

(54) **GENE MARKER COMBINATION, KIT, AND DETECTION METHOD**

(30) Priority: 11.09.2023 CN 202311584988
(71) Applicant: Acornmed Biotechnology Co., Ltd. Beijing, Beijing 101111 (CN); Tianjin Acornmed Medical Laboratory Co., Ltd., Tianjin 301799 (CN); Acornmed Medical Instrument Co., Ltd. Tianjin, Tianjin 301799 (CN)
(72) Inventor: LOU, Feng, Beijing 100000 (CN); CHEN, Libin, Beijing 100000 (CN); WANG, Wang, Beijing 100000 (CN); WANG, Huina, Beijing 100000 (CN); LIU, Lei, Beijing 100000 (CN); CAO, Shanbo, Beijing 100000 (CN)
(74) Representative: karo IP
(86) International application number: PCT/CN2024/082845
(87) International publication number: WO 2025/055298

(57) **Abstract**

The present application relates to the field of biomedicine, and in particular to a gene marker combination, a detection method, a kit and a detection model for urothelial carcinoma. According to the present application, a reagent combination is used for confirming the presence of urothelial carcinoma, assessing the risk of urothelial carcinoma and/or assessing the prognosis/progression of urothelial carcinoma. The reagent combination comprises reagents capable of detecting the methylation level of a DNA region or a fragment thereof where a VIM gene and a ONECUT2 gene are located in a sample under test, and gene mutations of TERT and FGFR3.

## Description

### Technical Field

The present application relates to the field of biomedicine, and in particular to a detection method, a kit and a detection model for urothelial carcinoma.

### Background Art

Among numerous cancers, the incidence of urinary system tumors is increasing year by year, thus the demand for accurate diagnosis and treatment of urinary system tumors is further expanding. Urothelial carcinoma (UC) comprises upper urinary tract urothelial carcinoma (UTUC) and lower urinary tract urothelial carcinoma (mainly bladder cancer (BC)), which have the characteristics of high incidence, multifocal primary tumors, easy recurrence after surgery, etc. At present, the commonly used methods for clinically detecting bladder cancer include cystoscopy (biopsy of suspected diseased tissues can be performed), urine exfoliative cytology test, imaging examination, and fluorescence in situ hybridization.

Clinical UC diagnosis and post-treatment monitoring mainly rely on imaging, urine testing, endoscopy, and biopsy. Traditional imaging examination is difficult for localization and qualitative diagnosis of small or morphologically atypical lesions. As an important diagnostic aids, urine testings are widely used in clinic, but they all have certain shortcomings. For example, both urine exfoliative cytology and FISH tests have limitations of relatively low sensitivity and specificity, which cannot replace microscopic examination and biopsy. Endoscopy (ureteroscopy/cystoscopy) combined with biopsy is a gold standard for the diagnosis and reexamination of urothelial tumors. However, due to its invasiveness and relatively high cost, patient compliance is poor, which makes it difficult to find early tumors and tumor recurrence in some patients, thus missing the opportunity of treatment.

In clinical practice, the early diagnosis and monitoring effect of UC are still poor. For example, the accuracy of CT examination of muscle-invasive bladder cancer (MIBC) is only 54.9%. Urine exfoliative cytology test and fluorescence in situ hybridization (FISH) test have low sensitivity and are easily affected by urinary system infection, bladder inflammation, calculi, and hematuria. Cystoscopy is the gold standard for the diagnosis of bladder cancer, but due to its invasive shortcomings, patients have psychological resistance, which makes the compliance of this method unguaranteed. Moreover, carcinoma in situ is difficult to be found under cystoscope and is easily confused with bladder inflammatory changes, thus biopsy is required for diagnosis.

Early diagnosis of upper urinary tract urothelial carcinoma (UTUC) still has great difficulties. When UTUC is highly suspected and can not be clarified by imaging examination, the tumor can be directly observed by ureteroscopy and biopsy can be performed. However, microscopic examination is affected by the location and angle of the tumor, resulting in conventional hard ureteroscopes often unable to enter the tumor site and unable to diagnose the tumor. Ureteral obstruction would also affect the results of microscopic examination, resulting in the inability to distinguish the causes of obstruction by microscopic examination. The causes of obstruction include ureteral calculi, stricture of pyeloureteral junction, and malignant ureteral tumors. Meanwhile, ureteroscopy itself also has certain problems. In addition to the risk of trauma and infection by ureteroscopy itself, the probability of exudation and adhesion formation of surrounding tissues after ureteroscopy is obviously increased and the difficulty of curative operation is increased. Therefore, ureteroscopy should not be carried out without careful consideration.

Therefore, it is necessary to provide a non-invasive detection product which has a high sensitivity and specificity and is capable of achieving accurate early diagnosis of and prognostic effects on urothelial carcinoma.

### Summary of the Invention

In order to improve the sensitivity and specificity of early diagnosis of urothelial carcinoma to achieve accurate early diagnosis and recurrence monitoring of urothelial carcinoma, the present application provides a method for confirming the presence of urothelial carcinoma, assessing the risk of urothelial carcinoma occurrence, and/or assessing the prognosis/progression of urothelial carcinoma, and a corresponding detection kit and/or detection model.

In the present application, specially selected mutation sites and methylated gene fragments are used as detection indexes to carry out early diagnosis and prognostic effect judgment of urothelial carcinoma. First of all, the detection method of the present application uses urine gDNA as a template and as an initial sample for detection. The method is a non-invasive detection method, which effectively avoids the occurrence of complications such as male urogenital infection, urethral and bladder bleeding, urethral injury, and urethral stricture, and is easy to implement, and thus can be detected at home. Secondly, conventional urine cytology test has an inadequate sensitivity, and a risk of false negative, especially the sensitivity for G1 grade (G1 grade, well-differentiated papillary carcinoma) and low-grade tumors is 16%. The detection kit for a simple methylation marker or a simple mutation marker has the risk of missed detection. The detection method of the present application employs a combined point mutation and methylation multi-omics analysis, effectively utilizes multiple information in a limited sample, can effectively identify especially early tumors, and has the advantages of high sensitivity and specificity. In addition, the detection method of the present application can obtain data results quickly, for example, by using a fluorescent quantitative PCR method, which facilitates patients to receive the subsequent treatment in time. The method, kit, and/or detection model of the present application have the advantages of high sensitivity and specificity.

In one aspect, the present application provides a method for confirming the presence of urothelial carcinoma, assessing the risk of urothelial carcinoma occurrence, and/or assessing the prognosis/progression of urothelial carcinoma, comprising detecting the methylation level of a DNA region or a fragment thereof where a VIM gene is located in a sample to be tested, and a gene mutation(s) in one or more genes selected from TERT and FGFR3.

In one aspect, the present application provides a method for confirming the presence of urothelial carcinoma, assessing the risk of urothelial carcinoma occurrence, and/or assessing the prognosis/progression of urothelial carcinoma, comprising detecting the methylation level of a DNA region or a fragment thereof where a VIM gene is located, and the methylation level of a DNA region or a fragment thereof where a ONECUT2 gene is located in a sample to be tested, and a gene mutation(s) in one or more genes selected from TERT and FGFR3. In some embodiments, the sample to be tested is from a tumor patient, a patient suspected of suffering from a tumor, a tumor susceptible population, a high-risk tumor population, or a healthy population.

In some embodiments, the tumor patient comprises a patient who has not been treated with an anti-cancer therapy and/or a patient who has been treated with an anti-cancer therapy.

In some embodiments, the sample to be tested comprises a tissue, a cell, and/or a body fluid.

In some embodiments, the sample to be tested comprises urine.

In some embodiments, the mutation(s) in the TERT gene comprise one or both selected from C250T and C228T. The point mutation TERT C250T mentioned in the present application is located 146 bp upstream of the ATG starting site of the TERT gene and may also be referred to as Chr5:1295250 C>T(GRCh37) or "c.-146C>T" in the art; and the point mutation TERT C228T is located 124 bp upstream of the ATG starting site of the TERT gene, and may also be referred to as Chr5:1295228 C >T(GRCh37) or "c.-124C>T" in the art.

In some embodiments, the mutation(s) in the FGFR3 gene comprise one or both selected from R248C and S249C. The point mutation FGFR3 R248C mentioned in the present application may also be referred to as Chr4: 1803564 C>T (GRCh37), or "NM_000142(FGFR3):c.742C>T (p.R248C)" in the art; and the point mutation FGFR3 S249C mentioned in the present application may also be referred to as Chr4: 1803568 C>G (GRCh37), or "NM_000142(FGFR3):c.746C>G (p.S249C)" in the art.

In some embodiments, the DNA region where the VIM gene is located comprises chr10:17270951-17271151, chr10:17271330-17271493, chr10:17271498-17271632, and/or chr10:17272405-17272436.

In some embodiments, the method comprises obtaining nucleic acids in the sample to be tested.

In some embodiments, the method comprises converting the nucleic acids such that the bases with methylation and the bases without methylation form different substances after the converting.

In some embodiments, the converting comprises converting by a deamination reagent and/or a methylation-sensitive restriction endonuclease.

In some embodiments, the deamination reagent comprises bisulfite.

In some embodiments, the method comprises contacting the sample to be tested with reagents capable of specifically recognizing and/or binding to the DNA region or the fragment thereof where the VIM gene is located and/or the gene mutation(s).

In some embodiments, the reagents comprise primers and/or a probe capable of specifically recognizing and/or binding to the DNA region or the fragment thereof where the VIM gene is located.

In some embodiments, the primers and/or the probe comprise the nucleic acid sequence as set forth in any one of SEQ ID NOs. 13-27.

In some embodiments, the method further comprises detecting the methylation level of a DNA region or a fragment thereof where a ONECUT2 gene is located in a sample to be tested.

In some embodiments, the DNA region where the ONECUT2 gene is located comprises chr18:55107803-55107828, chr18:55108590-55108672, chr18:55108794-55108826, and/or chr18:55109012-55109014.

In some embodiments, the method comprises contacting the sample to be tested with reagents capable of specifically recognizing and/or binding to the DNA region or the fragment thereof where the ONECUT2 gene is located.

In some embodiments, the reagents comprise primers and/or a probe capable of specifically recognizing and/or binding to the DNA region or the fragment thereof where the ONECUT2 gene is located.

In some embodiments, the primers and/or the probe comprise the nucleic acid sequence as set forth in any one of SEQ ID NOs. 1-12.

In some embodiments, the method comprises contacting the sample to be tested with reagents capable of specifically recognizing and/or binding to a mutation(s) in the TERT gene.

In some embodiments, the reagents comprise primers and/or a probe capable of specifically recognizing and/or binding to the mutation C250T in the TERT gene.

In some embodiments, the primers and/or the probe comprise the nucleic acid sequence as set forth in any one of SEQ ID NOs. 29-31.

In some embodiments, the reagents comprise primers and/or a probe capable of specifically recognizing and/or binding to the mutation C228T in the TERT gene.

In some embodiments, the primers and/or the probe comprise the nucleic acid sequence as set forth in any one of SEQ ID NOs. 28, 30, and 31.

In some embodiments, the method comprises contacting the sample to be tested with reagents capable of specifically recognizing and/or binding to a mutation(s) in the FGFR3 gene.

In some embodiments, the reagents comprise primers and/or a probe capable of specifically recognizing and/or binding to the mutation R248C in the FGFR3 gene.

In some embodiments, the primers and/or the probe comprise the nucleic acid sequence as set forth in any one of SEQ ID NOs. 32, 34, and 35.

In some embodiments, the reagents comprise the primers and/or the probe capable of specifically recognizing or binding to the mutation S249C in the FGFR3 gene.

In some embodiments, the primers and/or the probe comprise the nucleic acid sequence as set forth in any one of SEQ ID NOs. 33-35.

In some embodiments, the method comprises contacting the sample to be tested with reagents capable of specifically binding to a DNA region or a fragment thereof where a reference gene is located.

In some embodiments, the reference gene is an ACTB gene.

In some embodiments, the reagents capable of specifically binding to a DNA region or a fragment thereof where a reference gene is located comprise primers and/or a probe, and the primers and/or the probe comprise the nucleic acid sequence as set forth in any one of SEQ ID NOs. 39-44.

In some embodiments, the total concentration of the primers and/or the probe is 2-15 µM.

In some embodiments, the detection comprises using fluorescent PCR, and obtaining fluorescent Ct values. In some embodiments, the test result is considered to be positive if one or more methylation levels in the sample to be tested are detected to be positive, or one or more gene mutations are positive; and the test result is considered to be negative if all of the methylation levels in the sample to be tested are detected to be negative, and all of the gene mutations are negative.

In another aspect, the present application provides a reagent combination for confirming the presence of urothelial carcinoma, assessing the risk of urothelial carcinoma formation, and/or assessing the progression of urothelial carcinoma, wherein the reagent combination comprises reagents capable of detecting the methylation level of a DNA region or a fragment thereof where a VIM gene is located in a sample to be tested, and reagents capable of detecting a gene mutation(s) in one or more genes selected from TERT and FGFR3.

In another aspect, the present application provides a reagent combination for confirming the presence of urothelial carcinoma, assessing the risk of urothelial carcinoma formation, and/or assessing the progression of urothelial carcinoma, wherein the reagent combination comprises reagents capable of detecting the methylation level of a DNA region or a fragment thereof where a VIM gene is located, reagents capable of detecting the methylation level of a DNA region or a fragment thereof where a ONECUT2 gene is located in a sample to be tested, and reagents capable of detecting a gene mutation(s) in one or more genes selected from TERT and FGFR3.

In some embodiments, the sample to be tested is derived from a tumor patient, a patient suspected of suffering from a tumor, a tumor susceptible population, a high-risk tumor population, or a healthy population.

In some embodiments, the tumor patient comprises a patient who has not been treated with an anti-cancer therapy and/or a patient who has been treated with an anti-cancer therapy.

In some embodiments, the sample to be tested comprises a tissue, a cell, and/or a body fluid.

In some embodiments, the sample to be tested comprises urine.

In some embodiments, the DNA region where the VIM gene is located comprises chr10:17270951-17271151, chr10:17271330-17271493, chr10:17271498-17271632, and/or chr10:17272405-17272436.

In some embodiments, the reagents capable of detecting the methylation level of a DNA region or a fragment thereof where a VIM gene is located in a sample to be tested comprise primers and/or a probe capable of specifically recognizing and/or binding to the DNA region or the fragment thereof where the VIM gene is located.

In some embodiments, the primers and/or the probe capable of specifically recognizing and/or binding to a DNA region or a fragment thereof where a VIM gene is located comprise the nucleic acid sequence as set forth in any one of SEQ ID NOs. 13-27.

In some embodiments, the primers and/or the probe capable of specifically recognizing and/or binding to the DNA region or the fragment thereof where the VIM gene is located comprise any one selected from the following groups: (1) primers: SEQ ID NO. 13-14, probe: SEQ ID NO. 15; (2) primers: SEQ ID NOs. 16-17, probe: SEQ ID NO. 18; (3) primers: SEQ ID NOs. 19-20, probe: SEQ ID NO. 21; (4) primers: SEQ ID NOs. 22-23, probe: SEQ ID NO. 24; and (5) primers: SEQ ID NOs. 25-26, probe: SEQ ID NO. 27.

In some embodiments, the mutation(s) in the TERT gene comprise one or both selected from C250T and C228T.

In some embodiments, the reagent combination comprises primers and/or a probe capable of specifically recognizing and/or binding to the mutation C250T in the TERT gene.

In some embodiments, the primers and/or the probe capable of specifically recognizing and/or binding to the mutation C250T in the TERT gene comprise the nucleic acid sequence as set forth in any one of SEQ ID NOs. 29-31.

In some embodiments, the reagent combination comprises primers and/or a probe capable of specifically recognizing and/or binding to the mutation C228T in the TERT gene.

In some embodiments, the primers and/or the probe capable of specifically recognizing and/or binding to the mutation C228T in the TERT gene comprise the nucleic acid sequence as set forth in any one of SEQ ID NOs. 28, 30, and 31.

In some embodiments, the mutation(s) in the FGFR3 gene comprise one or both selected from R248C and S249C.

In some embodiments, the reagent combination comprises the primers and/or the probe capable of specifically recognizing and/or binding to the mutation R248C in the FGFR3 gene.

In some embodiments, the primers and/or the probe capable of specifically recognizing and/or binding to the mutation R248C in the FGFR3 gene comprise the nucleic acid sequence as set forth in any one of SEQ ID NOs. 32, 34, and 35.

In some embodiments, the reagent combination comprises the primers and/or the probe capable of specifically recognizing and/or binding to the mutation S249C in the FGFR3 gene.

In some embodiments, the primers and/or the probe capable of specifically recognizing and/or binding to the mutation S249C in the FGFR3 gene comprise the nucleic acid sequence as set forth in any one of SEQ ID NOs. 33-35.

In some embodiments, the reagent combination further comprises detecting the methylation level of a DNA region or a fragment thereof where a ONECUT2 gene is located in a sample to be tested.

In some embodiments, the DNA region where the ONECUT2 gene is located comprises chr18:55107803-55107828, chr18:55108590-55108672, chr18:55108794-55108826, and/or chr18:55109012-55109014.

In some embodiments, the reagents capable of detecting the methylation level of a DNA region or a fragment thereof where a ONECUT2 gene is located in a sample to be tested comprise primers and/or a probe capable of specifically recognizing and/or binding to the DNA region or the fragment thereof where the ONECUT2 gene is located.

In some embodiments, the primers and/or the probe capable of specifically recognizing and/or binding to the DNA region or the fragment thereof where the ONECUT2 gene is located comprise the nucleic acid sequence as set forth in any one of SEQ ID NOs. 1-12.

In some embodiments, the primers and/or the probe capable of specifically recognizing and/or binding to the DNA region or the fragment thereof where the ONECUT2 gene is located comprise any one selected from the following groups: (1) primers: SEQ ID NOs. 1-2, probe: SEQ ID NO. 3; (2) primers: SEQ ID NOs. 4-5, probe: SEQ ID NO. 6; (3) primers: SEQ ID NOs. 7-8, probe: SEQ ID NO. 9; (4) primers: SEQ ID NOs. 10-11, probe: SEQ ID NO. 12; and primers: SEQ ID NOs. 36-37, probe: SEQ ID NO. 38.

In some embodiments, the reagent combination further comprises reagents capable of specifically binding to a DNA region or a fragment thereof where a reference gene is located.

In some embodiments, the reference gene is an ACTB gene.

In some embodiments, the reagents capable of specifically binding to a DNA region or a fragment thereof where a reference gene is located comprise primers and/or a probe, and the primers and/or the probe comprise the nucleic acid sequence as set forth in any one of SEQ ID NOs. 39-44.

In some embodiments, the total concentration of the primers and/or the probe is 2-15 µM.

In some embodiments, the reagent combination further comprises a deamination reagent and/or a methylation-sensitive restriction endonuclease.

In some embodiments, the deamination reagent comprises bisulfite.

In another aspect, the present application provides the use of the reagent combination in the preparation of a kit.

In another aspect, the present application provides a kit comprising the reagent combination.

In another aspect, the present application provides a model for confirming the presence of urothelial carcinoma, assessing the risk of urothelial carcinoma occurrence, and/or assessing prognostic/progression of urothelial carcinoma, wherein the model is implemented on the basis of the kit.

Those skilled in the art can easily discern other aspects and advantages of the present application from the detailed description below. Only exemplary embodiments of the present application are shown and described in the following detailed description. As will be appreciated by those skilled in the art, the contents of the present application enable those skilled in the art to make changes to the specific embodiments disclosed without departing from the spirit and scope of the invention to which the present application relates. Accordingly, the description in the specification of the present application is merely exemplary and not limiting.

### Detailed Description of Embodiments

The implementation of the invention of the present application is illustrated by the specific embodiments below. Those familiar with this technology can easily understand other advantages and effects of the invention of the present application from the contents disclosed in the specification.

### Definition of Terms

In the present application, the term "VIM" generally refers to a gene encoding vimentin, which may also be referred to as CTRCT30 or HEL113. The human VIM gene is positioned in the forward strand of Chromosome 10: 17,270,258-17,279,592 (GRCh37:CM000672.1). For further information on human VIM gene, reference can be made to Ensemble NO. ENSG00000026025.

In the present application, the term "ONECUT2" generally refers to a gene encoding the transcription factor onecut family member 2. The human ONECUT2 gene is positioned in the forward strand of Chromosome 18: 55,102,917-55,158,529 (GRCh37:CM000680.1). For further information about human ONECUT2 gene, reference can be made to Ensemble NO. ENSG00000119547.

In the present application, the term "TERT" generally refers to a gene encoding telomerase reverse transcriptase, which may also be referred to as CMM9, DKCA2, DKCB4, EST2, PFBMFT1, TCS1, TP2, TRT, hEST2, or hTRT. The human TERT gene is positioned in the reverse strand of Chromosome 5: 1,253,262-1,295,184 (GRCh37:CM000667.1). For further information on human TERT gene, reference can be made to Ensemble NO. ENSG00000164362. Point mutations in the TERT gene involved in the present application can be expressed in one or more ways, for example, two common promoter mutations in the TERT gene in the present application are located 124 bp and 146 bp upstream of the ATG starting site, respectively, including Chr5:1295228 C>T (known as C228T, which may also be referred to as c.-124C>T in the art) and Chr5:1295250 C>T (known as C250T, which may also be referred to as c.-146C>T in the art).

In the present application, the term "FGFR3" generally refers to a gene encoding fibroblast growth factor receptor 3, which may also be referred to as ACH, CD333, CEK2, HSFGFR3EX, or JTK4. The human FGFR3 gene is positioned in the forward strand of Chromosome 4: 1,795,034-1,810,599 (GRCh37:CM000666.1). For further information about human FGFR3 gene, reference can be made to Ensemble NO. ENSG00000068078. Point mutations in the FGFR3 gene involved in the present application can be expressed in one or more ways, for example, the point mutation FGFR3 R248C mentioned in the present application may also be referred to as Chr4: 1803564 C>T (GRCh37), or "NM_000142(FGFR3):c.742C>T (p.R248C)" in the art; the point mutation FGFR3 S249C mentioned in the present application may also be referred to as Chr4: 1803568 C>G (GRCh37), or "NM_000142(FGFR3):c.746C>G (p.S249C)" in the art.

In the present application, the term "sample to be tested" generally refers to a sample that needs to be tested. For example, one or more gene regions on a sample to be tested may be detected for the presence of a modified state, or the presence of a mutation.

In the present application, the term "healthy population" generally refers to a population in whom a tumor is not currently detectable by a tumor detection method. A healthy population can develop into tumor patients, a high-risk tumor population, patients suspected of suffering from tumors, or a tumor susceptible population. "Healthy population" is a concept relative to "tumor patients", and "tumor patients" generally refer to a population who are confirmed to have tumors by pathological diagnosis and other detection methods.

In the present application, the term "high-risk tumor population" generally refers to a population at a relatively high risk of developing tumors. For example, a population with a family history of tumor diseases, a population with tumor susceptible genes, a population with unhealthy lifestyles (such as smoking, excessive alcohol consumption, often staying up late, etc.), and a population who are often exposed to toxic and harmful substances in their living or working environments.

In the present application, the term "patients suspected of suffering from tumors" generally refers to a population who have tumor symptoms but have not been confirmed to have tumors by pathological diagnosis or other detection methods.

In the present application, the term "tumor susceptible population" generally refers to a population with a susceptibility to tumor occurrence. For example, a population with a family history of tumor diseases, a population with tumor susceptible genes, a population with unhealthy lifestyles (such as smoking, excessive alcohol consumption, often staying up late, etc.), and a population who are often exposed to toxic and harmful substances in their living or working environments.

For urothelial carcinoma such as bladder cancer, the tumor susceptible population or high-risk tumor population may include, but are not limited to, the following populations: patients with unknown clinical diagnosis, and high-risk populations for urothelial carcinoma (individuals aged > 50 years; long-term smokers; a population with long-term exposure to carcinogens such as industrial chemical products; a population with family history of bladder cancer; and people who ingest aristolochic acid-containing Chinese herbal medicine).

In the present application, the term "DNA region" generally refers to a sequence of two or more naturally occurring or modified deoxyribonucleotides which are covalently bonded. For example, a DNA region of a gene can refer to the location of a specific deoxyribonucleotide sequence where a gene is located, for example, the deoxyribonucleotide sequence encodes the gene. For example, the DNA region of the present application includes the full length of the DNA region, a complementary region thereof, or a fragment of the foregoing regions. For example, a sequence at least about 20 kb upstream and downstream of the detection region provided in the present application can be used as a site for detection. For example, a sequence at least about 20 kb, at least about 15 kb, at least about 10 kb, at least about 5 kb, at least about 3 kb, at least about 2 kb, at least about 1 kb, or at least about 0.5 kb upstream and downstream of the detection region provided in the present application can be used as a site for detection. For example, suitable primers and probes can be designed according to the site, for used in methylation detection of a sample. In the present application, when a DNA region is mentioned or a base position is referred, it generally refers to the human reference genome version GRCh37, which corresponds to the version number hg19 of UCSC, unless otherwise specified.

In the present application, the term "methylation" generally refers to the methylation state of a gene fragment, a nucleotide, or a base thereof in the present application. For example, a DNA fragment where a gene is located in the present application can have a methylation modification(s) on one strand or both strands. For example, a DNA fragment where a gene is located in the present application can have a methylation modification(s) at one site or multiple sites. For example, methylation can refer to conversion of the cytosine at the 5' end of a dinucleotide of a CpG island on a DNA sequence to 5' methylcytosine (5mC), and can also include the formation of N6-methylpurine (N6-mA) and 7-methylguanine (7-mG).

In the present application, the term "conversion" generally refers to conversion of one or more structures into another structure(s). For example, the conversion in the present application may be specific. For example, a cytosine without a methylation modification can be converted to another structure (e.g., uracil) after conversion, and a cytosine with a methylation modification can remain substantially unchanged after conversion. For example, a cytosine without a methylation modification can be cleaved after conversion, and a cytosine with a methylation modification can remain substantially unchanged after conversion.

In the present application, the term "deamination reagent" generally refers to a substance having the ability to remove an amino group. For example, the deamination reagent can remove the amino group of an unmodified cytosine.

In the present application, the term "bisulfite" generally refers to a reagent that can distinguish between the DNA regions having and not having a modified state. For example, bisulfite may include a bisulfite, or an analog thereof, or a combination thereof. For example, bisulfite can deaminate the amino group of an unmodified cytosine to distinguish it from a modified cytosine. In the present application, the term "analog" generally refers to a substance having a similar structure and/or function. For example, an analog of bisulfite may have a similar structure to bisulfite. For example, an analog of bisulfite may refer to a reagent that can likewise distinguish between the DNA regions having and not having a modified state.

In the present application, the term "methylation sensitive restriction endonuclease" generally refers to an enzyme that selectively digests nucleic acids according to the methylation state of its recognition site. For example, for a restriction endonuclease that cleaves specifically when the recognition site is not methylated, cleavage may not occur or cleavage may occur with significantly reduced efficiency when the recognition site is methylated. For a restriction endonuclease that cleaves specifically when the recognition site is methylated, cleavage may not occur or cleavage may occur with significantly reduced efficiency when the recognition site is not methylated. For example, a methylation-specific restriction endonuclease can recognize a sequence containing a CG dinucleotide (e.g., cgcg or cccggg).

In the present application, the term "urothelial carcinoma" generally refers to a cancer associated with malignant proliferation of urothelial cells. Urothelial carcinoma may include upper urinary tract urothelial carcinoma (UTUC, e.g., renal pelvis cancer and ureter cancer) and lower urinary tract urothelial carcinoma (e.g., bladder cancer). Urothelial carcinoma can occur in the renal pelvis, ureter, or bladder.

In the present application, the term "progression" generally refers to a change of a disease from a less severe state to a more severe state. For example, tumor progression may include an increase in the number or severity of a tumor, the degree of metastasis of cancer cells, the rate of growth or spread of a cancer, and the like. For example, tumor progression may include progression of such a cancer from a stage with a less severe state to a stage with a more severe state, such as from stage I to stage II, from stage II to stage III, and the like.

In the present application, the term "occurrence" generally refers to the presence of a lesion in an individual. For example, when a tumor occurs, the individual can be diagnosed as a tumor patient.

In the present application, the term "fluorescent PCR" generally refers to a quantitative or semi-quantitative PCR technique. For example, it may be a PCR technique such as real-time quantitative polymerase chain reaction, quantitative polymerase chain reaction, or kinetic polymerase chain reaction. For example, the initial amount of target nucleic acid can be quantitatively detected using PCR amplification, and by means of an intercalating fluorescent dye, or a sequence-specific probe which can contain a fluorescent reporter molecule detectable only if it is hybridized to the target nucleic acid.

In the present application, the term "PCR amplification" generally refers to a polymerase chain amplification reaction. For example, PCR amplification in the present application may comprise any polymerase chain amplification reaction currently known to be used for DNA amplification.

In the present application, the term "fluorescence Ct value" generally refers to a measurement of a quantitative or semi-quantitative assessment of a target nucleic acid. For example, it may refer to the number of amplification reaction cycles experienced when the fluorescence signal reaches a preset threshold value.

### Detailed Description of the Invention

### Gene marker combination

The present application provides a gene marker combination which can be used to confirm the presence of urothelial carcinoma, assess the risk of urothelial carcinoma occurrence, and/or assess the prognosis/progression of urothelial carcinoma. Thereby, the present application provides a method for detecting and confirming the presence of urothelial carcinoma, assessing the risk of urothelial carcinoma occurrence, and/or assessing the prognosis/progression of urothelial carcinoma. The method can be used for early diagnosis and/or recurrence monitoring of urothelial carcinoma. The present application further provides a reagent combination for detecting the gene marker combination, and a kit comprising the reagent combination.

The inventors of the present application have found that by detecting the methylation level of the VIM gene in a sample to be tested and the point mutation(s) in the TERT gene, it is possible to confirm the presence of urothelial carcinoma, assess the risk of urothelial carcinoma occurrence, and/or assess the prognosis/progression of urothelial carcinoma with a high sensitivity and specificity.

In the present application, the gene marker combination comprises the methylation level of a DNA region or a fragment thereof where a VIM gene is located, and a gene mutation(s) of one or more genes selected from Table 2 below, for example, 1 mutation, 2 mutations, 3 mutations, 4 mutations, 5 or more mutations selected from the Table below. In some embodiments, the gene marker combination comprises the methylation level of a DNA region or a fragment thereof where a VIM gene is located, and a gene mutation(s) of one or more genes selected from the group of TERT, FGFR, and other gene mutations listed in Table 2 below, for example, 1 other mutation, 2 other mutations, 3 other mutations, 4 or more other mutations listed in Table 2 below selected from the Table below.

In the present application, the gene marker combination may comprise the methylation level of a DNA region or a fragment thereof where a VIM gene is located. In the present application, the DNA region where the VIM gene is located may be chr10: 17,270,258-17,279,592. For example, the DNA region where the VIM gene is located may be chr10:17,271,345-17,271,493, or a region thereof extended by about 100 bp in both upstream and downstream directions. For example, the DNA region where the VIM gene is located may be chr10: 17,271,051-17,271,051, or a region thereof extended by about 100 bp in both upstream and downstream directions. For example, the DNA region where the VIM gene is located may be chr10: 17,270,951-17,271,151, or a region thereof extended by about 100 bp in both upstream and downstream directions. For example, the DNA region where the VIM gene is located may be chr10: 17,272,405-17,272,436, or a region thereof extended by about 100 bp in both upstream and downstream directions. For example, the DNA region where the VIM gene is located may be chr10: 17,271,498-17,271,632, or a region thereof extended by about 100 bp in both upstream and downstream directions. The methylation level of the DNA region where the VIM gene is located can be detected so as to confirm the presence of urothelial carcinoma, assess the risk of urothelial carcinoma occurrence, and/or assess the prognosis/progression of urothelial carcinoma. If the methylation level of the DNA region where the VIM gene is located is positive, it can be considered that urothelial carcinoma is present, there is a high risk of urothelial carcinoma occurrence, and/or there is a high risk of urothelial carcinoma recurrence.

In the present application, the gene marker combination may comprise a mutation(s) in the TERT gene, for example, a mutation(s) in the TERT promoter. The mutation(s) in the TERT promoter may comprise a point mutation(s) located at C228 and/or C250. As an example, the mutation(s) in the TERT gene may comprise point mutation C228T. As another example, the mutation(s) in the TERT gene may comprise point mutation C250T. The presence of a mutation(s) in the TERT gene can be detected so as to confirm the presence of urothelial carcinoma, assess the risk of urothelial carcinoma occurrence, and/or assess the prognosis/progression of urothelial carcinoma. If a mutation(s) in the TERT gene is/are detected in a sample to be tested, in particular point mutation TERT C228T and/or TERT C250T, it can be considered that urothelial carcinoma is present, there is a high risk of urothelial carcinoma occurrence, and/or there is a high risk of urothelial carcinoma recurrence.

In the present application, the gene marker combination may comprise a mutation(s) in the FGFR3 gene, for example, a mutation(s) in the FGFR3 gene. The mutation(s) in the FGFR3 gene may comprise a gene mutation(s) that cause an amino acid mutation(s) of R248C and/or S249C. For example, the mutation(s) in the FGFR3 gene may comprise a point mutation that results in R248C, for example, point mutation C742T. For example, the mutation(s) in the FGFR3 gene may comprise a point mutation that results in S249C, e.g., point mutation C746G. The presence of a mutation(s) in the FGFR3 gene can be detected so as to confirm the presence of urothelial carcinoma, assess the risk of urothelial carcinoma occurrence, and/or assess the prognosis/progression of urothelial carcinoma. If a mutation(s) in the FGFR3 gene are detected in a sample to be tested, in particular the presence of R248C and/or S249C mutations, it can be considered that urothelial carcinoma is present, there is a high risk of urothelial carcinoma occurrence, and/or there is a high risk of urothelial carcinoma recurrence.

In the present application, the gene marker combination comprises the methylation level of a DNA region or a fragment thereof where a ONECUT2 gene is located. In the present application, the DNA region where the ONECUT2 gene is located may be chr 18: 55,102,917-55,158,529. For example, the DNA region where the ONECUT2 gene is located may be chr18: 55,107,803-55,107,828, or a region thereof extended by about 100 bp in both upstream and downstream directions. For example, the DNA region where the ONECUT2 gene is located may be chr18: 55,108,590-55,108,672, or a region thereof extended by about 100 bp in both upstream and downstream directions. For example, the DNA region where the ONECUT2 gene is located may be chr18: 55,109,012-55,109,014, or a region thereof extended by about 100 bp in both upstream and downstream directions. For example, the DNA region where the ONECUT2 gene is located may be chr18: 55,108,794-55,108,826, or a region thereof extended by about 100 bp in both upstream and downstream directions. The methylation level of the DNA region where a ONECUT2 gene is located can be detected so as to confirm the presence of urothelial carcinoma, assess the risk of urothelial carcinoma occurrence, and/or assess the prognosis/progression of urothelial carcinoma. If the methylation level of the DNA region where the ONECUT2 gene is located is positive, it can be considered that urothelial carcinoma is present, there is a high risk of urothelial carcinoma occurrence, and/or there is a high risk of urothelial carcinoma recurrence.

The present application confirms the presence of urothelial carcinoma, assesses the risk of urothelial carcinoma occurrence, and/or assesses the prognosis/progression of urothelial carcinoma by detecting the above gene marker combination.

In a specific embodiment, the gene marker combination may comprise the methylation level of the DNA region or the fragment thereof where the VIM gene is located, and one or more point mutations selected from the group of TERT C228T, TERT C250T, FGFR3 R248C, and FGFR3 S249C, for example, two, three, or four point mutations.

In a specific embodiment, the gene marker combination may comprise the methylation level of the DNA region or the fragment thereof where the VIM gene is located, and any one point mutation selected from the group of TERT C228T, TERT C250T, FGFR3 R248C, and FGFR3 S249C.

In a specific embodiment, the gene marker combination may comprise the methylation level of the DNA region or the fragment thereof where the VIM gene is located and a point mutation(s) in the TERT promoter.

In a specific embodiment, the gene marker combination may comprise the methylation level of the DNA region or the fragment thereof where the VIM gene is located and the point mutation TERT C228T.

In a specific embodiment, the gene marker combination may comprise the methylation level of the DNA region or the fragment thereof where the VIM gene is located and the point mutation TERT C250T.

In a specific embodiment, the gene marker combination may comprise the methylation level of the DNA region or the fragment thereof where the VIM gene is located and the point mutation FGFR3 R248C.

In a specific embodiment, the gene marker combination may comprise the methylation level of the DNA region or the fragment thereof where the VIM gene is located and the point mutation FGFR3 S249C.

In a specific embodiment, the gene marker combination may comprise the methylation level of the DNA region or the fragment thereof where the VIM gene is located, the point mutation(s) in FGFR3, and the point mutation(s) in FGFR3.

In a specific embodiment, the gene marker combination may comprise the methylation level of the DNA region or the fragment thereof where the VIM gene is located, and any two point mutations selected from the group of TERT C228T, TERT C250T, FGFR3 R248C, and FGFR3 S249C.

In a specific embodiment, the gene marker combination may comprise the methylation level of the DNA region or the fragment thereof where the VIM gene is located, the point mutation TERT C228T, and the point mutation FGFR3 S248C.

In a specific embodiment, the gene marker combination may comprise the methylation level of the DNA region or the fragment thereof where the VIM gene is located, the point mutation TERT C250T, and the point mutation FGFR3 R249C.

In a specific embodiment, the gene marker combination may comprise the methylation level of the DNA region or the fragment thereof where the VIM gene is located, and any three point mutations selected from the group of TERT C228T, TERT C250T, FGFR3 R248C, and FGFR3 S249C.

In a specific embodiment, the gene marker combination may comprise the methylation level of the DNA region or the fragment thereof where the VIM gene is located, and the following four point mutations: TERT C228T, TERT C250T, FGFR3 R248C, and FGFR3 S249C.

In a specific embodiment, the gene marker combination may comprise the methylation level of the DNA region or the fragment thereof where the VIM gene is located, the methylation level of the DNA region or the fragment thereof where the ONECUT2 gene is located, and any one or more of the following point mutations: TERT C228T, TERT C250T, FGFR3 R248C, and FGFR3 S249C, for example, two, three, or four point mutations.

In a specific embodiment, the gene marker combination may comprise the methylation level of the DNA region or the fragment thereof where the VIM gene is located, the methylation level of the DNA region or the fragment thereof where the ONECUT2 gene is located, and any one of the following point mutations: TERT C228T, TERT C250T, FGFR3 R248C, and FGFR3 S249C.

In a specific embodiment, the gene marker combination may comprise the methylation level of the DNA region or the fragment thereof where the VIM gene is located, the methylation level of the DNA region or the fragment thereof where the ONECUT2 gene is located, and TERT C228T.

In a specific embodiment, the gene marker combination may comprise the methylation level of the DNA region or the fragment thereof where the VIM gene is located, the methylation level of the DNA region or the fragment thereof where the ONECUT2 gene is located, and TERT C250T.

In a specific embodiment, the gene marker combination may comprise the methylation level of the DNA region or the fragment thereof where the VIM gene is located, the methylation level of the DNA region or the fragment thereof where the ONECUT2 gene is located, and FGFR3 R248C.

In a specific embodiment, the gene marker combination may comprise the methylation level of the DNA region or the fragment thereof where the VIM gene is located, the methylation level of the DNA region or the fragment thereof where the ONECUT2 gene is located, and FGFR3 S249C.

In a specific embodiment, the gene marker combination may comprise the methylation level of the DNA region or the fragment thereof where the VIM gene is located, the methylation level of the DNA region or the fragment thereof where the ONECUT2 gene is located, and any two of the following point mutations: TERT C228T, TERT C250T, FGFR3 R248C, and FGFR3 S249C.

In a specific embodiment, the gene marker combination may comprise the methylation level of the DNA region or the fragment thereof where the VIM gene is located, the methylation level of the DNA region or the fragment thereof where the ONECUT2 gene is located, and TERT C228T and FGFR3 R248C.

In a specific embodiment, the gene marker combination may comprise the methylation level of the DNA region or the fragment thereof where the VIM gene is located, the methylation level of the DNA region or the fragment thereof where the ONECUT2 gene is located, and TERT C250T and FGFR3 S249C.

In a specific embodiment, the gene marker combination may comprise the methylation level of the DNA region or the fragment thereof where the VIM gene is located, the methylation level of the DNA region or the fragment thereof where the ONECUT2 gene is located, and any three of the following point mutations: TERT C228T, TERT C250T, FGFR3 R248C, and FGFR3 S249C.

In a specific embodiment, the gene marker combination may comprise the methylation level of the DNA region or the fragment thereof where the VIM gene is located, the methylation level of the DNA region or the fragment thereof where the ONECUT2 gene is located, and the following four point mutations: TERT C228T, TERT C250T, FGFR3 R248C, and FGFR3 S249C.

The inventors of the present application have found that by detecting the methylation level of a VIM gene, the methylation level of a ONECUT2 gene, the point mutations C250T and C228T in TERT, and the point mutations R248C and S249C in FGFR3 in a sample to be tested, it is possible to confirm the presence of urothelial carcinoma, assess the risk of urothelial carcinoma occurrence, and/or assess the prognosis/progression of urothelial carcinoma with a high sensitivity and specificity.

In one embodiment, the reagent combination in the present application may comprise reagents for detecting the methylation level of the DNA region chr10:17271330-17271493 of VIM, reagents for detecting the methylation level of the DNA region chr18:55107803-55107828 of ONECUT2, reagents for detecting the point mutation TERT C228T, reagents for detecting the point mutation TERT C250T, reagents for detecting the point mutation FGFR3 R248C, and reagents for detecting the point mutation FGFR3 S249C.

### Gene marker detection method

Methods for detecting the methylation level of a DNA region or a fragment of a DNA region where a gene is located are well known to those skilled in the art and include, but are not limited to, methylation-specific PCR (MS-PCR), bisulfite conversion sequencing (BS-Seq), restriction endonuclease analysis in combination with sodium bisulfite, fluorescence quantification method, methylation-sensitive high-resolution melt curve analysis, pyrosequencing, chip-based methylation profiling, high-throughput sequencing, and flight mass spectrometry.

In a specific example, primer and probe sequences specific for the methylation of the gene to be tested can be designed to detect methylation levels by using fluorescence quantification methods based on the CpG island information of the gene to be tested (e.g., VIM or ONECUT2) in combination with fluorescence probe techniques. Primers and fluorescent dyes can also be used for detection.

For example, the methylation level of one or more DNA regions of ONECUT2 selected from the group of chr18:55107803-55107828, chr18:55108794-55108826, chr18:55108590-55108672, and chr18:55109012-55109014 can be detected. For example, the methylation level of at least one of the above DNA regions can be detected.

For example, the primers for detecting the methylation levels of the above DNA regions of ONECUT2 may comprise the nucleic acid sequence as set forth in SEQ ID NOs. 1-2, and the probe may comprise the nucleic acid sequence as set forth in SEQ ID NO. 3.

For example, the primers for detecting the methylation levels of the above DNA regions of ONECUT2 may comprise the nucleic acid sequence as set forth in SEQ ID NOs. 4-5, and the probe may comprise the nucleic acid sequence as set forth in SEQ ID NO. 6.

For example, the primers for detecting the methylation levels of the above DNA regions of ONECUT2 may comprise the nucleic acid sequence as set forth in SEQ ID NOs. 7-8, and the probe may comprise the nucleic acid sequence as set forth in SEQ ID NO. 9.

For example, the primers for detecting the methylation levels of the above DNA regions of ONECUT2 may comprise the nucleic acid sequence as set forth in SEQ ID NOs. 10-11, and the probe may comprise the nucleic acid sequence as set forth in SEQ ID NO. 12.

For example, the methylation levels of one or more DNA regions of VIM selected from the group of chr10: 17270951-17271151, chr10:17271330-17271493, chr10: 17271051-17271051, chr10: 17272405-17272436, and chr10: 17271498-17271632 are detected. For example, the methylation level of at least one of the above DNA regions can be detected.

For example, the primers for detecting the methylation levels of the above DNA regions of VIM may comprise the nucleic acid sequences as set forth in SEQ ID NOs. 13-14, and the probe may comprise the nucleic acid sequence as set forth in SEQ ID NO. 15.

For example, the primers for detecting the methylation levels of the above DNA regions of VIM may comprise the nucleic acid sequences as set forth in SEQ ID NOs. 16-17, and the probe may comprise the nucleic acid sequence as set forth in SEQ ID NO. 18.

For example, the primers for detecting the methylation levels of the above DNA regions of VIM may comprise the nucleic acid sequences as set forth in SEQ ID NOs. 19-20, and the probe may comprise the nucleic acid sequence as set forth in SEQ ID NO. 21.

For example, the primers for detecting the methylation levels of the above DNA regions of VIM may comprise the nucleic acid sequences as set forth in SEQ ID NOs. 22-23, and the probe may comprise the nucleic acid sequence as set forth in SEQ ID NO. 24.

For example, the primers for detecting the methylation levels of the above DNA regions of VIM may comprise the nucleic acid sequences as set forth in SEQ ID NOs. 25-26, and the probe may comprise the nucleic acid sequence as set forth in SEQ ID NO. 27.

Methods for detecting mutations or point mutations in genes of interest are well known to those skilled in the art, for example, detection methods based on PCR techniques, including but not limited to: PCR-SSCP, heteroduplex analysis (HA), mutant enrichment PCR, denaturing gradient gel electrophoresis (DGGE), chemical cleavage mismatch (CCM), allele-specific oligonucleotide (ASO) analysis, DNA chip ligase chain reaction (LCR), allele-specific amplification, RNase A cleavage, chromosomal in situ hybridization, fluorescence in situ hybridization, DNA sequence analysis, fluorescence quantitative PCR, digital PCR, and/or high throughput sequencing. For example, detection can be performed using fluorescent quantitative PCR depending on the primers involved in gene mutations, such as multiplex-specific primers. Detection can also be performed using primers and fluorescently labeled probes.

For example, the point mutation TERT C228T and/or the point mutation TERT C250T can be detected.

For example, the primers for detecting a point mutation in TERT may comprise the nucleic acid sequences as set forth in SEQ ID NOs. 28 and 30, and the probe may comprise the nucleic acid sequence as set forth in SEQ ID NO. 21.

For example, the primers for detecting a point mutation in TERT may comprise the nucleic acid sequences as set forth in SEQ ID NOs. 29-30, and the probe may comprise the nucleic acid sequence as set forth in SEQ ID NO. 21.

For example, the point mutation FGFR3 R248C and/or the point mutation FGFR3 S249C can be detected.

For example, the primers for detecting a point mutation in FGFR3 may comprise the nucleic acid sequences as set forth in SEQ ID NOs. 32 and 34, and the probe may comprise the nucleic acid sequence as set forth in SEQ ID NO. 35.

For example, the primers for detecting a point mutation in FGFR3 may comprise the nucleic acid sequences as set forth in SEQ ID NOs. 33-34, and the probe may comprise the nucleic acid sequence as set forth in SEQ ID NO. 35.

In the present application, the method further comprises one or more of the following steps: (1) obtaining nucleic acids (e.g., gDNAs) in a sample to be tested, (2) converting the nucleic acids with a deamination reagent (e.g., bisulfite), (3) adding the primer and/or probe combination described in the present application and the primer and/or probe combination of a reference gene, (4) performing real-time fluorescent quantitative PCR, and (5) obtaining the ΔCt values and analyzing the results. The ΔCt values may be: ΔCt value of a methylated fragment = Ct value of the methylated fragment - Ct value of the reference gene; and ΔCt value of a point mutation = Ct value of the point mutation - Ct value of the reference gene. The reference gene (which may also be referred to as an internal reference gene) may comprise a ACTB gene (β-actin), GAPDH, 18S rRNA, 28S rRNA, β2-MG, and PBGD. For example, the reference gene may be an ACTB gene.

In one embodiment, the primer and/or probe concentration for each gene marker in the combination may be the same or different. As an example, the concentration of the primer and/or probe mixture for a gene marker in the combination may be about 2-15 µM. As another example, the concentration of the primer and/or probe mixture for a gene marker in the combination may be about 6-12 µM. As another example, the concentration of the primer and/or probe mixture for a gene marker in the combination may be about 9 µM.

The present application includes detecting the combination of the above gene markers in a sample to be tested to confirm the presence of urothelial carcinoma, assess the risk of urothelial carcinoma occurrence, and/or assess the prognosis/progression of urothelial carcinoma. For example, according to the ΔCt value of the point mutation of each gene marker, if one or more in the above gene marker combination is (are) positive, the sample to be tested can be considered to be positive, and the subject from which the sample to be tested is derived can be considered to have urothelial carcinoma, a high risk of urothelial carcinoma occurrence, or a high risk of urothelial carcinoma recurrence. For example, a method for judging whether each gene marker is positive based on the ΔCt value may be that, when the ΔCt value of a methylated fragment is less than a certain value, the test result is considered to be positive and the target gene is judged to be hypermethylated, otherwise the target gene is hypomethylated; or when the ΔCt value of a point mutation is less than a certain value, the test result is considered to be positive and the site is judged to be mutated, otherwise the site is not mutated. For the values for ΔCt comparison, reference can be made to Table 1 below. Those skilled in the art can also select appropriate values for judgment according to actual needs.

**Table 1**

| Gene | △Ct | Judgment of gene detection results |
|---|---|---|
| ONECUT2 | ≤8 | Positive |
| VIM | ≤8 | Positive |
| FGFR3-R248C | ≤12 | Positive |
| TERT-C250T | ≤12 | Positive |
| FGFR3-S249C | ≤11 | Positive |
| TERT-C228T | ≤11 | Positive |

In the present application, a score value of the sample to be tested, such as a UC-Score value, may be calculated by a scoring model to judge whether the sample to be tested is negative or positive. If the sample's UC-Score is > 0, it is judged that the sample to be tested is positive; if UC-Score≦0, it is judged that the sample to be tested is negative. Those skilled in the art can obtain a suitable scoring model of UC-Score by analyzing the sample library data.

In another aspect, the present application provides one or more reagents or reagent combinations for detecting the gene marker combination. The one or more reagents may comprise primers and/or a probe.

In another aspect, the present application provides the use of the one or more reagents or reagent combinations in the preparation of a kit. The kit may comprise primers and/or a probe for detecting the gene marker combination, and optionally a pharmaceutically acceptable solvent, carrier, and/or instructions describing methods of use, and the like. In the kit, the primers and/or probe for detecting each of the gene markers, as well as other required reagents, may be placed in the same container or in different containers.

In another aspect, the present application provides a program capable of performing the method.

In another aspect, the present application provides the following embodiments:
1. A method for confirming the presence of urothelial carcinoma, assessing the risk of urothelial carcinoma occurrence, and/or assessing the prognosis/progression of urothelial carcinoma, comprising detecting the methylation level of a DNA region or a fragment thereof where a VIM gene is located in a sample to be tested, and a gene mutation(s) in one or more genes selected from TERT and FGFR3.
2. The method according to embodiment 1, wherein the sample to be tested is from a tumor patient, a patient suspected of suffering from a tumor, a tumor susceptible population, a high-risk tumor population, or a healthy population.
3. The method according to embodiment 2, wherein the tumor patient comprises a patient who has not been treated with an anti-cancer therapy and/or a patient who has been treated with an anti-cancer therapy.
4. The method according to any one of embodiments 1-3, wherein the sample to be tested comprises a tissue, a cell, and/or a body fluid.
5. The method according to any one of embodiments 1-4, wherein the sample to be tested comprises urine.
6. The method according to any one of embodiments 1-5, wherein the mutation(s) in the TERT gene comprise one or both selected from C250T and C228T.
7. The method according to any one of embodiments 1-6, wherein the mutation(s) in the FGFR3 gene comprise one or both selected from R248C and S249C.
8. The method according to any one of embodiments 1-7, wherein the DNA region where the VIM gene is located comprises chr10:17270951-17271151, chr10:17271330-17271493, chr10:17271498-17271632, and/or chr10:17272405-17272436.
9. The method according to any one of embodiments 1-8, wherein the method comprises obtaining nucleic acids in the sample to be tested.
10. The method according to embodiment 9, wherein the method comprises converting the nucleic acids such that bases with methylation and bases without methylation form different substances after the converting.
11. The method according to embodiment 10, wherein the converting comprises converting by a deamination reagent and/or a methylation-sensitive restriction endonuclease.
12. The method according to embodiment 11, wherein the deamination reagent comprises bisulfite.
13. The method according to any one of embodiments 1-12, wherein the method comprises contacting the sample to be tested with reagents capable of specifically recognizing and/or binding to the DNA region or the fragment thereof where the VIM gene is located and/or the gene mutation(s).
14. The method according to embodiment 13, wherein the reagents comprise primers and/or a probe capable of specifically recognizing and/or binding to the DNA region or the fragment thereof where the VIM gene is located.
15. The method according to embodiment 14, wherein the primers and/or the probe comprise the nucleic acid sequence as set forth in any one of SEQ ID NOs. 13-27.
16. The method according to any one of embodiments 1-13, further comprising detecting the methylation level of the DNA region or the fragment thereof where the ONECUT2 gene is located in a sample to be tested.
17. The method according to embodiment 16, wherein the DNA region where the ONECUT2 gene is located comprises chr18:55107803-55107828, chr18:55108590-55108672, chr18:55108794-55108826, and/or chr18:55109012-55109014.
18. The method according to embodiment 16 or 17, wherein the method comprises contacting the sample to be tested with reagents capable of specifically recognizing and/or binding to the DNA region or the fragment thereof where the ONECUT2 gene is located.
19. The method according to embodiment 18, wherein the reagents comprise primers and/or a probe capable of specifically recognizing and/or binding to the DNA region or the fragment thereof where the ONECUT2 gene is located.
20. The method according to embodiment 19, wherein the primers and/or the probe comprise the nucleic acid sequence as set forth in any one of SEQ ID NOs. 1-12.
21. The method according to any one of embodiments 1-20, wherein the method comprises contacting the sample to be tested with reagents capable of specifically recognizing and/or binding to the mutation(s) in the TERT gene.
22. The method according to embodiment 21, wherein the reagents comprise the primers and/or the probe capable of specifically recognizing and/or binding to the mutation C250T in the TERT gene.
23. The method according to embodiment 22, wherein the primers and/or the probe comprise the nucleic acid sequence as set forth in any one of SEQ ID NOs. 29-31.
24. The method according to embodiment 21, wherein the reagents comprise the primers and/or the probe capable of specifically recognizing and/or binding to the mutation C228T in the TERT gene.
25. The method according to embodiment 24, wherein the primers and/or the probe comprise the nucleic acid sequence as set forth in any one of SEQ ID NOs. 28, 30 and 31.
26. The method according to any one of embodiments 1-25, wherein the method comprises contacting the sample to be tested with reagents capable of specifically recognizing and/or binding to the mutation(s) in the FGFR3 gene.
27. The method according to embodiment 26, wherein the reagents comprise the primers and/or the probe capable of specifically recognizing and/or binding to the mutation R248C in the FGFR3 gene.
28. The method according to embodiment 27, wherein the primers and/or the probe comprise the nucleic acid sequence as set forth in any one of SEQ ID NOs. 32, 34 and 35.
29. The method according to embodiment 26, wherein the reagents comprise the primers and/or the probe capable of specifically recognizing or binding to the mutation S249C in the FGFR3 gene.
30. The method according to embodiment 29, wherein the primers and/or the probe comprise the nucleic acid sequence as set forth in any one of SEQ ID NOs. 33-35.
31. The method according to any one of embodiments 1-30, further comprising contacting the sample to be tested with reagents capable of specifically binding to a DNA region or a fragment thereof where a reference gene is located.
32. The method according to embodiment 31, wherein the reference gene is an ACTB gene.
33. The method according to embodiment 31 or 32, wherein the reagents capable of specifically binding to the DNA region or the fragment thereof where the reference gene is located comprise primers and/or a probe, and the primers and/or the probe comprise the nucleic acid sequence as set forth in any one of SEQ ID NOs. 39-44.
34. The method according to any one of embodiments 1-33, wherein the detecting comprises using fluorescent PCR, and obtaining fluorescent Ct values.
35. The method according to any one of embodiments 1-34, further comprising: the test result is considered to be positive if one or more methylation levels in the sample to be tested are detected to be positive, or one or more gene mutations are positive; and the test result is considered to be negative if all of the methylation levels in the sample to be tested are detected to be negative, and all of the gene mutations are negative.
36. A reagent combination for confirming the presence of urothelial carcinoma, assessing the risk of urothelial carcinoma formation, and/or assessing the progression of urothelial carcinoma, wherein the reagent combination comprises reagents capable of detecting the methylation level of a DNA region or a fragment thereof where a VIM gene is located in a sample to be tested, and reagents capable of detecting a gene mutation(s) in one or more genes selected from TERT and FGFR3.
37. The reagent combination according to embodiment 36, wherein the sample to be tested is from a tumor patient, a patient suspected of suffering from a tumor, a tumor susceptible population, a high-risk tumor population, or a healthy population.
38. The reagent combination according to embodiment 37, wherein the tumor patient comprises a patient who has not been treated with an anti-cancer therapy and/or a patient who has been treated with an anti-cancer therapy.
39. The reagent combination according to any one of embodiments 36-38, wherein the sample to be tested comprises a tissue, a cell, and/or a body fluid.
40. The reagent combination according to any one of embodiments 36-39, wherein the sample to be tested comprises urine.
41. The reagent combination according to any one of embodiments 36-40, wherein the DNA region where the VIM gene is located comprises chr10:17270951-17271151, chr10:17271330-17271493, chr10:17271498-17271632, and/or chr10:17272405-17272436.
42. The reagent combination according to any one of embodiments 36-41, wherein the reagents capable of detecting the methylation level of the DNA region or the fragment thereof where the VIM gene is located in the sample to be tested comprise primers and/or a probe capable of specifically recognizing and/or binding to the DNA region or the fragment thereof where the VIM gene is located.
43. The reagent combination according to embodiment 42, wherein the primers and/or the probe capable of specifically recognizing and/or binding to the DNA region or the fragment thereof where the VIM gene is located comprise the nucleic acid sequence as set forth in any one of SEQ ID NOs. 13-27.
44. The reagent combination according to embodiment 42 or 43, wherein the primers and/or the probe capable of specifically recognizing and/or binding to the DNA region or the fragment thereof where the VIM gene is located comprise the nucleic acid sequences selected from any one of the following groups:
   (1) primers: SEQ ID NOs. 13-14, probe: SEQ ID NO. 15;
   (2) primers: SEQ ID NOs. 16-17, probe: SEQ ID NO. 18;
   (3) primers: SEQ ID NOs. 19-20, probe: SEQ ID NO. 21;
   (4) primers: SEQ ID NOs. 22-23, probe: SEQ ID NO. 24; and
   (5) primers: SEQ ID NOs. 25-26, probe: SEQ ID NO. 27.
45. The reagent combination according to any one of embodiments 36-44, wherein the mutation(s) in the TERT gene comprise one or both selected from C250T and C228T.
46. The reagent combination according to embodiment 45, comprising the primers and/or the probe capable of specifically recognizing and/or binding to the mutation C250T in the TERT gene.
47. The reagent combination according to embodiment 46, wherein the primers and/or the probe capable of specifically recognizing and/or binding to the mutation C250T in the TERT gene comprise the nucleic acid sequence as set forth in any one of SEQ ID NOs. 29-31.
48. The reagent combination according to any one of embodiments 36-47, comprising the primers and/or the probe capable of specifically recognizing and/or binding to the mutation C228T in the TERT gene.
49. The reagent combination according to embodiment 48, wherein the primers and/or the probe capable of specifically recognizing and/or binding to the mutation C228T in the TERT gene comprise the nucleic acid sequence as set forth in any one of SEQ ID NOs. 28, 30, and 31.
50. The reagent combination according to any one of embodiments 36-49, wherein the mutation(s) in the FGFR3 gene comprise one or both selected from R248C and S249C.
51. The reagent combination according to embodiment 50, comprising the primers and/or the probe capable of specifically recognizing and/or binding to the mutation R248C in the FGFR3 gene.
52. The reagent combination according to embodiment 51, wherein the primers and/or the probe capable of specifically recognizing and/or binding to the mutation R248C in the FGFR3 gene comprise the nucleic acid sequence as set forth in any one of SEQ ID NOs. 32, 34, and 35.
53. The reagent combination according to any one of embodiment 50, comprising the primers and/or the probe capable of specifically recognizing and/or binding to the mutation S249C in the FGFR3 gene.
54. The reagent combination according to embodiment 53, wherein the primers and/or the probe capable of specifically recognizing and/or binding to the mutation S249C in the FGFR3 gene comprise the nucleic acid sequence as set forth in any one of SEQ ID NOs. 33-35.
55. The reagent combination according to any one of embodiments 36-54, further comprising detecting the methylation level of the DNA region or the fragment thereof where the ONECUT2 gene is located in the sample to be tested.
56. The reagents according to embodiment 55, wherein the DNA region where the ONECUT2 gene is located comprises chr18:55107803-55107828, chr18:55108590-55108672, chr18:55108794-55108826, and/or chr18:55109012-55109014.
57. The reagent combination according to embodiment 55 or 56, wherein the reagents capable of detecting the methylation level of the DNA region or the fragment thereof where the ONECUT2 gene is located in the sample to be tested comprise primers and/or a probe capable of specifically recognizing and/or binding to the DNA region or the fragment thereof where the ONECUT2 gene is located.
58. The reagent combination according to embodiment 57, wherein the primers and/or the probe capable of specifically recognizing and/or binding to the DNA region or the fragment thereof where the ONECUT2 gene is located comprise the nucleic acid sequence as set forth in any one of SEQ ID NOs. 1-12.
59. The reagent combination according to any one of embodiments 55-58, wherein the primers and/or the probe capable of specifically recognizing and/or binding to the DNA region or the fragment thereof where the ONECUT2 gene is located comprise the nucleic acid sequences selected from any one of the following groups:
   (1) primers: SEQ ID NOs. 1-2, probe: SEQ ID NO. 3;
   (2) primers: SEQ ID NOs. 4-5, probe: SEQ ID NO. 6;
   (3) primers: SEQ ID NOs. 7-8, probe: SEQ ID NO. 9; and
   (4) primers: SEQ ID NOs. 10-11, probe: SEQ ID NO. 12.
60. The reagent combination according to any one of embodiments 36-59, further comprising reagents capable of specifically binding to a DNA region or a fragment thereof where a reference gene is located.
61. The reagent combination according to embodiment 60, wherein the reference gene is an ACTB gene.
62. The reagent combination according to embodiment 60 or 61, wherein the reagents capable of specifically binding to the DNA region or the fragment thereof where the reference gene is located comprise primers and/or a probe, and the primers and/or the probe comprise the nucleic acid sequence as set forth in any one of SEQ ID NOs. 39-44.
63. The reagent combination according to any one of embodiments 36-62, further comprising a deamination reagent and/or a methylation-sensitive restriction endonuclease.
64. The reagent combination according to embodiment 63, wherein the deamination reagent comprises bisulfite.
65. Use of the reagent combination according to any one of embodiments 37-64 in the preparation of a kit.
66. A kit comprising the reagent combination according to any one of embodiments 37-64.
67. A model for confirming the presence of urothelial carcinoma, assessing the risk of urothelial carcinoma occurrence, and/or assessing prognosis/progression of urothelial carcinoma, wherein the model is implemented on the basis of the kit according to embodiment 66.

**Table 2**

| Number | Gene | Chromosome | Base mutation form | Amino acid mutation form | Frequency of mutation in a tumor population |
|---|---|---|---|---|---|
| 1 | ASXL2 | 2 | c.988G>A | p.Glu330Lys | 1% |
| 2 | SF3B1 | 2 | c.2704G>C | p.Glu902Gln | 1% |
| 3 | | 2 | c.2704G>A | p.Glu902Lys | |
| 4 | RHOA | 3 | c.145G>A | p.Asp49Asn | 1% |
| 5 | | 3 | c.139G>A | p.Glu47Lys | |
| 6 | | 3 | c.278G>A | p.Arg93Gln | |
| 7 | | 3 | c.316G>C | p.Gly106Arg | |
| 8 | | 3 | c.317G>T | p.Gly106Val | |
| 9 | PIK3CA | 3 | c.323G>A | p.Arg108His | |
| 10 | | 3 | c.1357G>A | p.Glu453Lys | 16% |
| 11 | | 3 | c.1624G>A | p.Glu542Lys | |
| 12 | | 3 | c.1625A>T | p.Glu542Val | |
| 13 | | 3 | c.1633G>A | p.Glu545Lys | |
| 14 | | 3 | c.1633G>C | p.Glu545GIn | |
| 15 | | 3 | c.1634A>G | p.Glu545Gly | |
| 16 | | 3 | c.1636C>A | p.Gln546Lys | |
| 17 | | 3 | c.2176G>A | p.Glu726Lys | |
| 18 | | 3 | c.3139C>T | p.His1047Tyr | |
| 19 | | 3 | c.3140A>G | p.His1047Arg | |
| 20 | | 3 | c.3140A>T | p.His1047Lcu | |
| 21 | | 4 | c.742C>T | p.Arg248Cys | |
| 22 | | 4 | c.746C>G | p.Ser249Cys | |
| 23 | | 4 | c.749C>A | p.Pro250GIn | |
| 24 | | 4 | c.1102G>T | p.Glu368Ter | |
| 25 | | 4 | c.1111_1112insAC C | p.Ser371Cys | |
| 26 | | 4 | c.1111A>T | p.Ser371Cys | |
| 27 | FGFR3 | 4 | c.1118A>G | p.Tyr373Cys | 34% |
| 28 | | 4 | c.1127T>C | p.Ile376Thr | |
| 29 | | 4 | c.1138G>A | p.Gly380Arg | |
| 30 | | 4 | c.1271C>T | p.Ser424Phe | |
| 31 | | 4 | c.1277A>G | p.Glu426Gly | |
| 32 | | 4 | c.1280C>G | p.Ser427Cys | |
| 33 | FBXW7 | 4 | c.1637C>T | p.Ser546Leu | 1% |
| 34 | | 4 | c.1595C>T | p.Thr532Ile | |
| 35 | | 4 | c.1594A>C | p.Thr532Pro | |
| 36 | | 4 | c.1394G>A | p.Arg465His | |
| | | | | | |
| | | | | | |
| | | | | | |
| | | | | | |
| | | | | | |
| | | | | | |
| | | | | | |
| | | | | | |
| 37 | TERT | 5 | c.-45G>T | / | 50% |
| 38 | | 5 | c.-54C>A | / | |
| 39 | | 5 | c.-57A>C | / | |
| 40 | | 5 | c.-63T>CATTT | / | |
| 41 | | 5 | c.-80C>T | / | |
| 42 | | 5 | c.-86C>T | / | |
| 43 | | 5 | c.- 89C>T | / | |
| 44 | | 5 | c.-93T>G | / | |
| 45 | | 5 | c.-124C>T | / | |
| 46 | | 5 | c.-124C>A | / | |
| 47 | | 5 | c.-124_-125GG>AA | / | |
| 48 | | 5 | c.-138C>T | / | |
| 49 | | 5 | c.-139_-140GG>TT | / | |
| 50 | | 5 | c.-139C>T | / | |
| 51 | | 5 | c.-141G>A | / | |
| 52 | | 5 | c.-146C>T | / | |
| 53 | HRAS | 11 | c.218G>A | p.Arg73His | 8% |
| 54 | | 11 | c.182A>T | p.Gln61Leu | |
| 55 | | 11 | c.182A>G | p.Gln61Arg | |
| 56 | | 11 | c.38G>A | p.Gly13Asp | |
| 57 | | 11 | c.37G>C | p.Gly13Arg | |
| 58 | | 11 | c.35G>A | p.Gly12Asp | |
| 59 | | 11 | c.34G>A | p.Gly12Ser | |
| 60 | | 11 | c.19G>C | p.Val7Leu | |
| 61 | KRAS | 12 | C.76A>T | p.Asn26Tyr | 1% |
| 62 | | 12 | c.57G>C | p.Leu19Phe | |
| 63 | | 12 | c.38G>A | p.Gly13Asp | |
| 64 | | 12 | c.35G>T | p.Gly12Val | |
| 65 | | 12 | c.35G>C | p.Gly12Ala | |
| 66 | | 12 | c.35G>A | p.Gly12Asp | |
| 67 | | 12 | c.34G>C | p.Gly12Arg | |
| 68 | ERBB3 | 12 | c.889G>T | p.Asp297Tyr | 1% |
| 69 | | 12 | c.994G>A | p.Glu332Lys | |
| 70 | | 12 | c.1009G>A | p.Gly337Arg | |
| 71 | AKT1 | 14 | c.49G>A | p.Glu17Lys | 1% |
| 72 | CREBBP | 16 | c.3217C>T | p.GIn1073Ter | 11% |
| 73 | ERBB2 | 17 | c.291G>C | p.Gln97His | 8% |
| 74 | | 17 | c.308G>A | p.Arg103Gln | |
| 75 | | 17 | c.829G>C | p.Asp277His | |
| 76 | | 17 | c.829G>T | p.Asp277Tyr | |
| 77 | | 17 | c.874G>C | p.Gly292Arg | |
| 78 | | 17 | c.914C>G | p.Ser305Cys | |
| 79 | | 17 | c.929C>A | p.Ser310Tyr | |
| 80 | | 17 | c.929C>T | p.Ser310Phe | |
| 81 | | 17 | c.937C>G | p.Leu313Val | |
| 82 | | 17 | c.1979G>A | p.Gly660Asp | |
| 83 | | 17 | c.2183C>T | p.Ser728Phe | |
| 84 | | 17 | c.2324_2325ins12 | / | |
| 85 | ERCC2 | 19 | c.713A>G | p.Asn238Ser | 2% |
| 86 | | 19 | c.705G>T | p.Glu235Asp | |
| 87 | | 19 | c.702C>A | P.Asp234Glu | |
| 88 | U2AF1 | 21 | c.101C>T | p.Ser34Phe | 1% |
| 89 | | 21 | c.101C>A | p.Ser34Tyr | |
| 90 | KDM6A | X | c.1870delC | / | 23% |
| 91 | | X | c.1896G>A | p.Trp632Ter | |
| 92 | | X | c.1906_1909deICT AT | / | |
| 93 | | X | c.1909_1912delTC TA | / | |
| 94 | | X | c.1910_1911insT | / | |
| 95 | | X | c.1916_1917insTA | / | |
| 96 | | X | c.1921C>T | p.Gln641Ter | |
| 97 | TP53 | 17 | Exonic SNP mutation | / | 31% |

Without intending to be limited by any theory, the following examples are only intended to set forth the gene marker combination, reagent combination, kit, detection methods and use, etc., of the present application, and are not intended to limit the scope of the invention of the present application.

### Examples

The primer and probe sequences used in the Examples of the present application are shown in Table 3 below:

**Table 3**

| Gene marker | Name | Sequence number (SEQ ID NO) | Primer sequence (5' to 3') | 5' modification | 3' modification |
|---|---|---|---|---|---|
| ONECUT2 methylation sites 1-4: chr18:55107803-55107828, chr18:55108590-55108672, chr18:55108794-55108826, and chr18:55109012-55109014 | ONECUT2-F1 | 1 | GTTTTTGTGYGTATATATYGTT ATTTGYGTT | - | - |
| | ONECUT2-R1 | 2 | CRCATTAACTTCRCRAACCCAA C | - | - |
| | ONECUT2-P1 | 3 | TTCGGCGATTCGTTTGGGCG | 5'6-FAM | 3'BHQ1 |
| | ONECUT2-F2 | 4 | TGGTAGTAAGTTTTTAGYGGTT AGTTTGA | | |
| | ONECUT2-R2 | 5 | TCAAATAACCCAAACRACATCA TAATAAATTAAC | | |
| | ONECUT2-P2 | 6 | TAGGCGGTCGAGGGTT | 5'VIC | 3'MGB |
| | ONECUT2-F3 | 7 | GGYGYGTTTTTTTTAGTAGAGY GTT | | |
| | ONECUT2-R3 | 8 | AAAACTAACRAAACRACACCR CTAA | | |
| | ONECUT2-P3 | 9 | AGCGTTACGGTTTCGCGGTTTT | 5'6-FAM | 3'BHQ1 |
| | ONECUT2-F4 | 10 | GTTAAYGGTAGAGTTTAATTA GTYGTAGATTT | | |
| | ONECUT2-R4 | 11 | AAACCACRTTTAATTATCCAAC RAATACA | | |
| | ONECUT2-P4 | 12 | TTACGCGTAGATTTTTTACGTA G | 5'VIC | 3'MGB |
| VIM methylation sites 1-4: chr10:17271330-17271493, chr10: 17270951-17271151, chr10: 17272405-17272436, and | VIM-1-F1 | 13 | GGTTYGTGTTTTYGTTTTTTTAT YGTAGGAT | - | - |
| | VIM-1-R1 | 14 | ACRTAATCACRTAACTCCRACT AAAACTC | - | - |
| | VIM-1-P1 | 15 | TTCGGCGGTTCGGGTATCGC | 5'CY5 | 3'BHQ3 |
| | VIM-1-F2 | 16 | GTTTTYGTTYGTTTTTTTTTYGG GAGTTAG | | |
| | VIM-1-R2 | 17 | CRAAAACACRAACCTAATAAA CATAACTAC | | |
| chr10: 17271498-17271632 | VIM-1-P2 | 18 | TTCGCGTTATCGTCGTCGTTTA GGTTA | 5'CY5 | 3'BHQ3 |
| | VIM-2-F1 | 19 | TTTTTAGTATTTTAGGGTGAGT TTAGTTTAGATTAT | | |
| | VIM-2-R1 | 20 | CCTAAAACTAAACATAATCCCG TTACTTCA | | |
| | VIM-2-P1 | 21 | ATTCGGAAAGTTTTTAAAAGTT TTAG | 5'CY5 | 3'BHQ3 |
| | VIM-3-F1 | 22 | GAGGGAGCGAGATAAAGGGA TG | | |
| | VIM-3-R1 | 23 | CACRATAACCRCACAAACCTAC AA | | |
| | VIM-3-P1 | 24 | TTCGGGGGCGGGGATGG | 5'CY5 | 3'BHQ3 |
| | VIM-4-F1 | 25 | GGTYGAGTTTTAGTYGGAGTT AYGTGATTA | | |
| | VIM-4-R1 | 26 | CACRACAAAAAAACRCRTAAC ATACAC | | |
| | VIM-4-P1 | 27 | TTACGTTTCGTTTTCGGGCGGC | 5'CY5 | 3'BHQ3 |
| Point mutations C228T and C250T in TERT | TERT-M2-F | 28 | GCTGGGAGGGCCAGGAA | - | - |
| | TERT-M1-F | 29 | GGGCCGGGGACCCTGA | - | - |
| | TERT-R | 30 | CTCCCAGTGGATTCGCGG | - | - |
| | TERT-P | 31 | CCACGTGGGAAGCGCGGTC | 5'6-FAM | 3'BHQ1 |
| Point mutations R248C and S249C in FGFR3 | FGFR3-M1-F | 32 | CATCTGCCCCCACAGGGT | - | - |
| | FGFR3-M2-F | 33 | GCCCCCACAGAGCGGTG | - | - |
| | FGFR3-R | 34 | TTCACCTCCACGTGCTTGAG | - | - |
| | FGFR3-P | 35 | TGGGCAGCGACGTGGAGTTCC | 5'CY5 | 3'BHQ2 |
| ONECUT2 methylation site | ONECUT2-F5 | 36 | ATTTTTGTATTATTCGTTTTTGT GCGTATA | | |
| | ONECUT2-R5 | 37 | GCGTTTTCGGCGATTCGTTTGG GCGGTT | | |
| | ONECUT2-P5 | 38 | CCGTTCAACGCATTAACTTCGC GA | 5'6-FAM | 3'BHQ1 |
| ACTB reference gene | ACTB-F | 39 | GGTTGTGTTTAGTTAATGGG | | |
| | ACTB-R | 40 | AATCATCTTTCCCACCAAAC | | |
| | ACTB-P | 41 | TATAACCTCTACAACCTTCAAA A | 5'VIC | 3'MGB |
| | ACTB-M1-M2-F | 42 | GTCAGGCAGCTCGTAGCTCT | - | - |
| | ACTB-M1-M2-R | 43 | ATCGTGCGTGACATTAAGGAG | - | - |
| | ACTB-M1-M2-P | 44 | CCGTGGCCATCTCTTGCTCGAA GT | 5'VIC | 3'BHQ1 |
| EOMES methylation site chr3:27765233-27765233 | EOMES-F1 | 45 | GTTTTTTCGTTGCGTTTTTTTAG ATAGATT | | |
| | EOMES-R1 | 46 | AAACRTACCCCCCRCTTCC | | |
| | EOMES-P1 | 47 | AAGAGCGGGGTCGTTATATTT TATCGTTT | 5'VIC | 3'BHQ1 |
| Point mutation chr177577085_757 7085_C>T in TP53 | TP53-F2 | 48 | CTTTCTTGCGGAGATTCTCTTC CTC | | |
| | TP53-R3 | 49 | AATGGGACAGGTAGGACCTG | | |
| | TP53-P1 | 50 | CTCTCCCAGGACAGGCACAAA C | 5'VIC | 3'BHQ1 |
| TWIST1 methylation site chr7:19157242-19157264 | TWIST1-F1 | 51 | GGAAAYGGTGTYGGTGTTGTA GA | | |
| | TWIST1-R1 | 52 | TACACRAAAATATAAAAACCTC CAAATCTAC | | |
| | TWIST1-P1 | 53 | TTCGCGAGGTGTTTGGGAGTT G | 5'6-FAM | 3'BHQ1 |
| Point mutation chr10: 115511593 C T in PLEKHS1 | PLEKHS1-593-F10 | 54 | TGATCTAGAAGCTTTTTTGCAA TTAAAT | | |
| | PLEKHS1-90-93-R1 | 55 | TGAATACAGTATACCAAGAGG TCAGTCC | | |
| | PLEKHS1-90-93-P1 | 56 | TGCTGTATTTCTGTTATGGGCA C | 5'6-FAM | 3'BHQ1 |

In the sequence, Y represents C or T, and R represents A or G.

### Example 1. Non-Diagnostic Detection Method for Urothelial Carcinoma

S1. gDNAs were extracted from urine samples and subjected to bisulfite conversion.

### 1. Extraction of gDNAs from urinary sediment

The extraction of gDNAs from urinary sediment was performed with a cell extraction kit which was purchased from Zymo with a product model of Quick-DNA Urine Kit (D3061). The specific steps were as follows:

Urine pretreatment: the collected 50 ml urine sample was centrifuged at 1,600 g for 10 min. After centrifugation was completed, the supernatant was discarded. 1 ml PBS buffer was added, pipetted and mixed thoroughly. The mixture was transferred to a 1.5 ml centrifuge tube. A 1.5 ml tube containing the urinary sediment was centrifuged at 12,000 g for 1 min, and subsequently the waste liquid was discarded. 1 ml of PBS buffer was added to rinse the sediment obtained in 1.2 once. Centrifugation was performed at 12,000 g for 1 min, and subsequently the waste liquid was discarded. The sediment was washed again once, and the waste liquid was discarded. 200 ul GA buffer (a GA buffer which was used to suspend cells) was directly added to a 1.5 ml centrifuge tube containing the sediment, pipetted and mixed thoroughly. Then 20 ul protease K was added, and mixed thoroughly under shaking. Then 200 ul GB buffer (which was used for cell lysis and separation of nucleic acids and proteins) was added, mixed thoroughly under shaking, and the mixture was centrifuged transiently. Subsequently, incubation was performed at 70°C for 10 min. After the incubation was completed, 200 ul of anhydrous ethanol was added, and mixed thoroughly under shaking for 15 s. The solution was added to a CB3 column, centrifugation was performed at 12,000 g for 1 min, and the waste liquid was discarded. Then, 500 µl of GD rinse solution (a rinse solution which was used to remove protein impurities) was added to the CB3 column, centrifugation was performed at 12,000 rpm for 30 s, and the waste liquid was discarded. Subsequently, 600 µl PW (a rinse solution which was used for salt removal) was added to the CB3 column, centrifugation was performed at 12,000 rpm for 1 min, and the waste liquid was discarded. This process was repeated once. Centrifugation was performed at 12,000 rpm for 2 min, the waste liquid was discarded, and the residual liquid around the column was aspirated. Subsequently, the CB3 column was placed at room temperature for 5 min and air-dried. The above CB3 column was transferred into a clean 1.5 ml tube, and 53 µl TE (which was used for dissolving DNAs) was added dropwise onto the column membrane, followed by placing at room temperature for 5 min. Centrifugation was performed at 12,000 g for 2 min and the waste liquid was discarded.

### 2. Conversion of gDNAs in urinary sediment by sulfite

Conversion of gDNAs in urinary sediment by sulfite was performed with a Zymo kit, which was purchased from Tianmo Biotech with a product model of EZ DNA Methylation-Lightning Kit (D3051). The specific steps were as follows:
80 ng of gDNAs obtained in Step 1 was taken, and nuclease-free water (NFW) was added to a volume of 20 ul. 130 ul of Lightning Conversion Reagent was added, mixed thoroughly under shaking, and reaction was performed under the following reaction conditions: reacting at 98°C for 8 min, subsequently reacting at 54°C for 60 min, and maintaining at 4°C. 600 µl of binding buffer (M-Binding Buffer) was added to the Zymo-Spin^{™} IC column, and all of the above incubated solution was transferred to the Zymo-Spin^{™} IC. The column was inverted 10 times to mix thoroughly. Then centrifugation was performed at 10,000 g for 1 min and the waste liquid was discarded. 100 ul of washing buffer (M-Wash Buffer) was added to the Zymo-Spin^{™} IC column, subsequently centrifugation was performed at 10,000 g for 1 min, and the waste liquid was discarded. Then, 200 ul of L-Desulphonation Buffer was added to the Zymo-Spin^{™} IC column, followed by standing at room temperature for 15 min. Subsequently, centrifugation was performed at 10,000 g for 30 s, and the waste liquid was discarded. Next, 200 ul of washing buffer (M-Wash Buffer) was added to the Zymo-Spin^{™} IC column, centrifugation was performed at 10,000 g for 1 min, and the waste liquid was discarded. This process was repeated once. The resulting Zymo-Spin^{™} IC was placed in a new 1.5 ml centrifuge tube, 10 µl of elution buffer (M-Elution Buffer) was added, and then centrifugation was performed at 10,000 g for 30 s. Samples were collected and quantitatively detected using Qubit^{™} ssDNA detection kit.

S2. The sulfite-converted gDNA was used as a template, and was mixed with the primer and probe set for the methylated gene fragments, the primer and probe set of the point mutation sites, and the primer and probe set of the control gene, for performing the real-time fluorescent quantitative PCR.

The reaction mixture was prepared on ice according to the formulation scheme of 12 µl detection reaction mix, 9 µl probe and primer mix, and 30 ng template (in order to ensure the accuracy of reaction solution preparation, when performing each reaction, the detection reaction mix and the probe and primer mix should be prepared according to the amounts required for the reaction number plus 2, then aliquoted into each reaction tube, and finally the converted DNA samples were added). While a sample to be tested was added, a positive quality control, a negative quality control, and a negative sample were added to judge whether the experiments were qualified at this time. The reaction conditions are shown in Table 4.

**Table 4. PCR reaction conditions**

| Stage | Temperature | Time | Cycle number | Fluorescence signal acquisition |
|---|---|---|---|---|
| 1 | 37°C | 3min | 1 | No |
| 2 | 98°C | 2min | 1 | No |
| 3 | 98°C | 20s | 45 | No |
| 4 | 62°C | 45s | | Yes |

S3. The data results were analyzed.

After the reaction was completed, the data were read and analyzed by using the dedicated software for real-time fluorescent quantitative PCR instrument (i.e., Real-Time PCR Instrument 7500). The ΔCt value was obtained for each methylated gene fragment and mutation site, respectively.

The ΔCt value was read according to the following method:
I. Reading the PCR reaction results of the probe and primer mixture for methylation:
   (1) Baseline setting: the baseline was set manually, and the start cycle and the end cycle of the baseline were generally set at 3 and 15, respectively. (2) Threshold setting: the threshold was set manually and was generally set at the exponential phase of PCR amplification, which was the inflection point where the amplification curve rose. (3) Ct value determination: after the baseline and fluorescence threshold were set, the "Analysis" key was clicked to obtain the Ct value of each sample.

ΔCt value of a methylated fragment = Ct value of the methylated fragment - Ct value of the reference gene.

According to Table 1 above, when the ΔCt value of the methylated fragment is ≤ 8, it is judged to be positive, and the target gene is hypermethylated, otherwise the target gene is hypomethylated.

II. Reading the results of PCR reaction mixture containing the probe and primers for point mutations: (1) Baseline setting: the baseline was set manually, and the start cycle and the end cycle of the ACTB-M baseline was generally set at 3 and 15, respectively. (2) Fluorescence threshold setting: the threshold was set manually and was generally set at the exponential phase of PCR amplification, which was the inflection point where the amplification curve rose. (3) Ct value determination: after the baseline and fluorescence threshold were set, the "Analysis" key was clicked to obtain the Ct value of each sample.

ΔCt value of a point mutation = Ct value of the point mutation - Ct value of the reference gene.

According to Table 1 above, when the ΔCt value of the point mutation is ≤11 or 12, it is judged that there is a mutation at the site, otherwise there is no mutation at the site.

### Example 2. Test Results of Two-Gene-Markers in Early Diagnosis of Urothelial Carcinoma

252 patients suspected of suffering from urothelial carcinoma were enrolled (urine samples were collected from patients with microscopic or gross hematuria without a history of malignant disease in the last five years. All patients in the pathologically positive group were pathologically confirmed by endoscopy, abdominal ultrasonic sound, CT scan, or abdominal and pelvis MRI examinations. For patients in the gold standard negative group, patients were excluded from the diagnosis of urothelial tumors after endoscopic examination and imaging evaluation), and were tested using the detection procedures of Example 1, respectively. After the experiments were completed, analysis was performed using the software of the instrument. The threshold value was adjusted to be above the baseline fluorescence value, so that no Ct value appeared in the negative control. The samples' Ct values calculated by the automatic analysis of the instrument were recorded. A detection result was considered below the detection limit if the Ct value was not detected or exceeded 45, and such value was converted to 45. Then ΔCt was calculated (ΔCt = Ct of the test gene - Ct of the internal reference gene ACTB).

Criteria for judgment: a gene marker combination includes a gene mutation and a methylation. If either or both of the gene mutation and methylation are positive, the test result is judged to be positive for a patient. For a gene marker combination including a mutation(s) at one or more gene sites, if the mutation(s) at one or two sites is/are determined to be positive, the gene mutation of the gene marker combination is determined to be positive.

It was compared with the test results of the gold standard cystoscopic pathology, in which the test results of cystoscopic pathology were positive in 131 cases and negative in 121 cases. The gene marker combinations used are shown in Table 5, and the test results are shown in Table 6-1 to Table 6-5, and Table 7. The results show that compared with the control group, both the detection sensitivity and specificity of the experimental groups were relatively high, and were ≥ 80%.

**Table 5. Two-gene-marker combinations**

| Group | Gene marker combination | Primer and probe combination |
|---|---|---|
| Experimental group 1 | VIM methylation site 1 + TERT C228T | VIM-1-F1, VIM-1-R1, VIM-1-P1, TERT-M2-F, TERT-R, and TERT-P |
| Experimental group 2 | VIM methylation site 2 + TERT C228T | VIM-2-F1, VIM-2-R1, VIM-2-P1, TERT-M2-F, TERT-R, and TERT-P |
| Experimental group 3 | VIM methylation site 3 + TERT C228T | VIM-3-F1, VIM-3-R1, VIM-3-P1, TERT-M2-F, TERT-R, and TERT-P |
| Control group 1 | EOMES methylation + TERT C228T | EOMES-F1, EOMES-R1, EOMES-P1, TERT-M2-F, TERT-R, and TERT-P |
| Control group 2 | VIM methylation site 2 + TP53: chr17 7577085_7577085_C>T | VIM-2-F1, VIM-2-R1, VIM-2-P1, TP53-F2, TP53-R3, and TP53-P1 |

**Table 6-1. Test results of the experimental group 1**

| Experimental group | | Pathological diagnosis | |
|---|---|---|---|
| | | Positive | Negative |
| Method of the present application | Positive | 110 | 7 |
| | Negative | 21 | 114 |

**Table 6-2. Test results of the experimental group 2**

| Experimental group | | Pathological diagnosis | |
|---|---|---|---|
| | | Positive | Negative |
| Method of the present application | Positive | 108 | 7 |
| | Negative | 23 | 114 |

**Table 6-3. Test results of the experimental group 3**

| Control group | | Pathological diagnosis | |
|---|---|---|---|
| | | Positive | Negative |
| Method of the present application | Positive | 109 | 5 |
| | Negative | 22 | 116 |

**Table 6-4. Test results of the control group 1**

| Control group | | Pathological diagnosis | |
|---|---|---|---|
| | | Positive | Negative |
| Method of the present application | Positive | 55 | 20 |
| | Negative | 76 | 101 |

**Table 6-5. Test results of the control group 2**

| Control group | | Pathological diagnosis | |
|---|---|---|---|
| | | Positive | Negative |
| Method of the present application | Positive | 60 | 34 |
| | Negative | 71 | 87 |

**Table 7. Summary of the test results of two-gene combinations used for early diagnosis**

| Group | Gene marker combination | Sensitivity | Specificity |
|---|---|---|---|
| Experimental group 1 | VIM methylation site 1 + TERT C228T | 83.9% | 94.2% |
| Experimental group 2 | VIM methylation site 2 + TERT C228T | 82.4% | 94.2% |
| Experimental group 3 | VIM methylation site 3 + TERT C228T | 83.2% | 95.8% |
| Control group 1 | EOMES methylation + TERT C228T | 41.9% | 84.3% |
| Control group 2 | VIM methylation site 2 + TP53: chr17 7577085_7577085_C>T | 45.8% | 71.9% |

### Example 3. Test Results of Two-Gene-Markers in Recurrence Monitoring of Urothelial Carcinoma

47 patients suspected of urothelial carcinoma recurrence were enrolled, and detected by the method of Example 2, respectively, and the detection results were compared with those of the gold standard cystoscopic pathological examination. The gene marker combinations used are shown in Table 5, and the test results are shown in Table 8-1, Table 8-2, Table 8-3, Table 8-4, and Table 9. The results show that compared with the control group, both the detection sensitivity and specificity of the experimental groups were relatively high, and were ≥ 80%.

**Table 8-1. Test results of the experimental group 1**

| Experimental group | | Pathological diagnosis | |
|---|---|---|---|
| | | Positive | Negative |
| Method of the present application | Positive | 22 | 4 |
| | Negative | 2 | 19 |

**Table 8-2. Test results of the experimental group 2**

| Experimental group | | Pathological diagnosis | |
|---|---|---|---|
| | | Positive | Negative |
| Method of the present application | Positive | 21 | 4 |
| | Negative | 3 | 19 |

**Table 8-3. Test results of the control group 1**

| Control group | | Pathological diagnosis | |
|---|---|---|---|
| | | Positive | Negative |
| Control method | Positive | 14 | 8 |
| | Negative | 10 | 15 |

**Table 8-4. Test results of the control group 2**

| Control group | | Pathological diagnosis | |
|---|---|---|---|
| | | Positive | Negative |
| Control method | Positive | 11 | 5 |
| | Negative | 13 | 18 |

**Table 9. Summary of the test results of two-gene combinations used for recurrence monitoring**

| Group | Gene marker combination | Sensitivity | Specificity |
|---|---|---|---|
| Experimental group 1 | VIM methylation site 1 + TERT C228T | 91.6% | 82.6% |
| Experimental group 2 | VIM methylation site 2 + TERT C228T | 87.5% | 82.6% |
| Control group 1 | EOMES methylation + TERT C228T | 58.3% | 65.2% |
| Control group 2 | VIM methylation site 2 + TP53: chr17 7577085_7577085_C>T | 45.5% | 78.3% |

### Example 4. Test Results of Three-Gene-Markers in Early Diagnosis of Urothelial Carcinoma

252 patients suspected of suffering from urothelial carcinoma were enrolled (urine samples were collected from patients with microscopic or gross hematuria without a history of malignant disease in the last five years. All patients in the pathologically positive group were pathologically confirmed by endoscopy, abdominal ultrasonic sound, CT scan, or abdominal and pelvis MRI examinations. For patients in the gold standard negative group, patients were excluded from the diagnosis of urothelial tumors after endoscopic examination and imaging evaluation), and were tested using the detection procedures of Example 1, respectively. After the experiments were completed, analysis was performed using the software of the instrument. The threshold value was adjusted to be above the baseline fluorescence value, so that no Ct value appeared in the negative control. The samples' Ct values calculated by the automatic analysis of the instrument were recorded. A detection result was considered below the detection limit if the Ct value was not detected or exceeded 45, and such value was converted to 45. Then ΔCt was calculated (ΔCt = Ct of the test gene - Ct of the internal reference gene ACTB).

Criteria for judgment: a gene marker combination includes a gene mutation and a methylation. If either or both of the gene mutation and methylation are positive, the test result is judged to be positive for a patient. For a gene marker combination including a mutation(s) at one or more gene sites, if the mutation(s) at one or two sites is/are determined to be positive, the gene mutation of the gene marker combination is determined to be positive.

It was compared with the test results of the gold standard cystoscopic pathology, in which the test results of cystoscopic pathology were positive in 131 cases and negative in 121 cases. The gene marker combinations used are shown in Table 10, and the test results are shown in Table 11-1, Table 11-2, and Table 12. The results show that both the detection sensitivity and specificity of the experimental group 1 and experimental group 2 were relatively high, and were ≥70%, with the experimental group 1 achieving ≥85%.

**Table 10. Three-gene-marker combinations**

| Group | Gene marker combination | Primer and probe combination |
|---|---|---|
| Experimental group 1 | VIM methylation site 1 + TERT C228T + FGFR3 R248C | VIM-1-F1, VIM-1-R1, VIM-1-P1, TERT-M2-F, TERT-R, TERT-P, FGFR3-M1-F, FGFR3-R, and FGFR3-F |
| Experimental group 2 | ONECUT2 methylation + TERT C228T + TERT C250T | ONECUT2-F5, ONECUT2-R5, ONECUT2 P5, TERT-M2-F, TERT-R, TERT-P, TERT-M1-F, TERT-R, and TERT-P |

**Table 11-1. Test results of the experimental group 1**

| Experimental group | | Pathological diagnosis | |
|---|---|---|---|
| | | Positive | Negative |
| Method of the present application | Positive | 115 | 8 |
| | Negative | 16 | 113 |

**Table 11-2. Test results of the experimental group 2**

| Experimental group | | Pathological diagnosis | |
|---|---|---|---|
| | | Positive | Negative |
| Method of the present application | Positive | 98 | 22 |
| | Negative | 33 | 99 |

**Table 12. Summary of test results of three-gene combinations for early diagnosis**

| Group | Gene marker combination | Sensitivity | Specificity |
|---|---|---|---|
| Experimental group 1 | VIM methylation site 1 + TERT C228T + FGFR3 R248C | 87.7% | 93.3% |
| Experimental group 2 | ONECUT2 methylation + TERT C228T + TERT C250T | 74.8% | 81.8% |

### Example 5. Test Results of Six-Gene-Markers in Early Diagnosis of Urothelial Carcinoma

252 patients suspected of suffering from urothelial carcinoma were enrolled (urine samples were collected from patients with microscopic or gross hematuria without a history of malignant disease in the last five years. All patients in the pathologically positive group were pathologically confirmed by endoscopy, abdominal ultrasonic sound, CT scan, or abdominal and pelvis MRI examinations. For patients in the gold standard negative group, patients were excluded from the diagnosis of urothelial tumors after endoscopic examination and imaging evaluation), and were tested using the detection procedures of Example 1, respectively. After the experiments were completed, analysis was performed using the software of the instrument. The threshold value was adjusted to be above the baseline fluorescence value, so that no Ct value appeared in the negative control. The samples' Ct values calculated by the automatic analysis of the instrument were recorded. A detection result was considered below the detection limit if the Ct value was not detected or exceeded 45, and such value was converted to 45. Then ΔCt was calculated (ΔCt = Ct of the test gene - Ct of the internal reference gene ACTB).

Criteria for judgment: a gene marker combination includes a gene mutation and a methylation. If either or both of the gene mutation and methylation are positive, the test result is judged to be positive for a patient. For a gene marker combination including a mutation(s) at one or more gene sites, if the mutation(s) at one or two sites is/are determined to be positive, the gene mutation of the gene marker combination is determined to be positive.

It was compared with the test results of the gold standard cystoscopic pathology, in which the test results of cystoscopic pathology were positive in 131 cases and negative in 121 cases. The gene marker combinations used are shown in Table 13, and the test results are shown in Table 14-1, Table 14-2, and Table 15. The results show that compared with the control group, both the detection sensitivity and specificity of the experimental group 1 were relatively high, and were ≥90%, while the sensitivity and specificity of the control group were ≤70%.

**Table 13. Six-gene-marker combinations**

| Group | Gene marker combination | Primer and probe combination |
|---|---|---|
| Experimental group 1 | VIM methylation site 1 + ONECUT2 methylation site 1 + TERT C228T + TERT C250T + FGFR3 S249C + FGFR3 R248C | VIM-1-F1, VIM-1-R1, VIM-1-P1, ONECUT2-F1, ONECUT2-R1, ONECUT2-P1, TERT-M2-F, TERT-R, TERT-P, FGFR3-M1-F, FGFR3-R, and FGFR3-F |
| Control group 1 | TWIST1 methylation + ONECUT2 methylation site 1 + TERT C250T + TERT C228T + TP53: chr17 7577085_7577085_C>T + PLEKHS1_chr10_115511593_C>T | TWIST1-F1, TWIST1-R1, TWIST1-P1, ONECUT2-F1, ONECUT2-R1, ONECUT2-P1, TERT-M2-F, TERT-R, TERT-P, TERT-M1-F, TERT-R, TERT-P, TP53-F2, TP53-R3, TP53-P1, PLEKHS1-593-F10, PLEKHS1-90-93-R1, and PLEKHS1-90-93-P1 |

**Table 14-1. Test results of the experimental group 1**

| Experimental group | | Pathological diagnosis | |
|---|---|---|---|
| | | Positive | Negative |
| Method of the present application | Positive | 119 | 8 |
| | Negative | 12 | 113 |

**Table 14-2. Test results of the control group 1**

| Control group | | Pathological diagnosis | |
|---|---|---|---|
| | | Positive | Negative |
| Control method | Positive | 87 | 42 |
| | Negative | 44 | 79 |

**Table 15. Summary of test results of six-gene combinations for early diagnosis**

| Group | Gene marker combination | Sensitivity | Specificity |
|---|---|---|---|
| Experimental group 1 | VIM methylation site 1 + ONECUT2 methylation site 1 + TERT C228T + TERT C250T + FGFR3 S249C + FGFR3 R248C | 90.8% | 93.4% |
| Experimental group 2 | TWIST1 methylation + ONECUT2 methylation site 1 + TERT C250T + TERT C228T + TP53: chr17 7577085_7577085_C> T + PLEKHS1_chr10_115511593_C>T | 66.4% | 65.2% |

### Example 6. Test Results of Six-Gene-Markers in Recurrence Monitoring of Urothelial Carcinoma

47 patients suspected of urothelial carcinoma recurrence were enrolled, and detected by the method of Example 5, respectively, and the detection results were compared with those of the gold standard cystoscopic pathological examination. The gene marker combinations used are shown in Table 13, and the test results are shown in Table 16-1, Table 16-2, and Table 17. The results show that compared with the control group, both the detection sensitivity and specificity of the experimental groups were relatively high, and were ≥ 90%, while those of the control groups were less than 60%.

**Table 16-1. Test results of the experimental group 1**

| Experimental group | | Pathological diagnosis | |
|---|---|---|---|
| | | Positive | Negative |
| Method of the present application | Positive | 23 | 2 |
| | Negative | 1 | 21 |

**Table 16-2. Test results of the control group 1**

| Control group | | Pathological diagnosis | |
|---|---|---|---|
| | | Positive | Negative |
| Control method | Positive | 17 | 8 |
| | Negative | 7 | 15 |

**Table 17. Summary of test results of six-gene combinations for early diagnosis**

| Group | Gene marker combination | Sensitivity | Specificity |
|---|---|---|---|
| Experimental group 1 | VIM methylation site 1 + ONECUT2 methylation site 1 + TERT | 95.8% | 91.3% |
| | C228T + TERT C250T + FGFR3 S249C + FGFR3 R248C | | |
| Experimental group 2 | TWIST1 methylation + ONECUT2 methylation site 1 + TERT | 41.3% | 55.6% |
| | C250T + TERT C228T + TP53: chr17 7577085_7577085_C>T + PLEKHS1_chr10_115511593_C>T | | |

### Example 7. Test Results of Different Primer/Probe Sequences for Six-Gene Combinations

According to mutiple methylated regions of VIM or ONECUT2, different primer and probe combinations were designed, and the urine samples from 252 patients suspected of suffering from urothelial carcinoma and 47 patients with recurrence were tested according to the method of Example 5 or Example 6. The primer and probe combinations for the six-gene-marker combinations were as described in Example 5, except that only the primers and probes for VIM or ONECUT2 methylations were replaced with the primers and probes in Table 18. The test results are shown in Table 19. The results show that both the detection sensitivity and specificity of the six-gene combinations were relatively high for different methylated fragments, all of which were ≥ 85%.

**Table 18**

| Methylated gene | Methylated fragment region | Group | Primers and probe |
|---|---|---|---|
| VIM | VIM methylation site 2 | Group 1 | VIM-2-F1, VIM-2-R1, and VIM-2-P1 |
| | VIM methylation site 1 | Group 2A | VIM-1-F1, VIM-1-R1, and VIM-1-P1 (sequences in Example 5) |
| | VIM methylation site 1 | Group 2B | VIM-1-F2, VIM-1-R2, and VIM-1-P2 |
| | VIM methylation site 4 | Group 3 | VIM-4-F1, VIM-4-R1, and VIM-4-P1 |
| | VIM methylation site 3 | Group 4 | VIM-3-F1, VIM-3-R1, and VIM-3-P1 |
| ONECUT2 | ONECUT2 methylation site 2 | Group 1 | ONECUT2-F2, ONECUT2-R2, and ONECUT2-P2 |
| | ONECUT2 methylation site 3 | Group 2 | ONECUT2-F3, ONECUT2-R3, and ONECUT2-P3 |
| | ONECUT2 methylation site 1 | Group 3 | ONECUT2-F1, ONECUT2-R1, and ONECUT2-P1 (sequences in Example 5) |
| | ONECUT2 methylation site 4 | Group 4 | ONECUT2-F4, ONECUT2-R4, and ONECUT2-P4 |

**Table 19**

| Group | Experimental group 1 (sequences in Example 5) | Experimental group 2 | Experimental group 3 | Experimental group 4 | Experimental group 5 | Experimental group 6 | Experimental group 7 | Experimental group 8 |
|---|---|---|---|---|---|---|---|---|
| Primer pair | VIM group 2A + ONECUT2 group 3 | The marker replacing the one in experimental group 1 | | | | | | |
| | | VIM group 1 | VIM group 2B | VIM group 3 | VIM group 4 | ONECUT2 group 1 | ONECUT2 group 2 | ONECUT2 group 4 |
| Sensitivi ty | 90.8% (119/131) | 90.1% (118/131) | 89.3% (117/131) | 88.5% (116/131) | 87.8% (115/131) | 87.0% (114/131) | 89.3% (117/131) | 88.5% (116/131) |
| Specifici ty | 93.4% (113/121) | 87.6% (106/121) | 90.1% (109/121) | 89.3% (108/121) | 87.6% (106/121) | 91.7% (111/121) | 90.1% (109/121) | 93.4% (113/121) |

### Example 8. Test Results of Six-Gene-Markers in Early Diagnosis of Urothelial Carcinoma

529 patients suspected of suffering from urothelial carcinoma were enrolled (296 patients were pathologically diagnosed as positive and 233 patients were pathologically diagnosed as negative), and were tested using the method of Example 5. The gene marker combinations used are shown in Table 13. The test results are shown in Table 20. The results show that both the detection sensitivity and specificity of the six-gene test experimental group were relatively high, and were ≥ 85%.

**Table 20. Test results of experimental groups**

| Experimental group | | Pathological diagnosis | |
|---|---|---|---|
| | | Positive | Negative |
| Method of the present application | Positive | 258 | 33 |
| | Negative | 38 | 200 |

The sensitivity and specificity of the test results in the present application were: sensitivity = 258/296 = 87.2%, specificity = 200/233 = 85.8%, respectively.

## Claims

1. Use of a reagent combination in the preparation of a kit, wherein the reagent combination comprises: a reagent capable of detecting the methylation level of a DNA region or a fragment thereof where the VIM gene is located, and a reagent capable of detecting a gene mutation(s) in one or more genes selected from TERT and FGFR3 in a sample to be tested, and the kit is for confirming the presence of urothelial carcinoma, assessing the risk of urothelial carcinoma occurrence, and/or assessing the progression of urothelial carcinoma, and the DNA region where the VIM gene is located comprises any one of the following groups: chr10:17270851-17271251, chr10:17271245-17271593, chr10:17271398-17271732, and chr10:17272305-17272536.

2. The use according to claim 1, wherein the sample to be tested is derived from a tumor patient, a patient suspected of suffering from a tumor, a tumor susceptible population, a high-risk tumor population, or a healthy population.

3. The use according to claim 2, wherein the tumor patient includes a patient untreated with anti-cancer therapy and/or a patient treated with anti-cancer therapy.

4. The use according to any one of claims 1-3, wherein the sample to be tested comprises a tissue, a cell, and/or a body fluid.

5. The use according to any one of claims 1-4, wherein the sample to be tested comprises urine.

6. The use according to any one of claims 1-5, wherein the reagent capable of detecting the methylation level of the DNA region or a fragment thereof where the VIM gene in the sample to be tested comprise a primer and/or a probe capable of specifically recognizing and/or binding to said DNA region or the fragment thereof where the VIM gene is located.

7. The use according to claim 6, wherein the primer and/or the probe capable of specifically recognizing and/or binding to said DNA region or the fragment thereof where the VIM gene is located comprises the nucleic acid sequence as set forth in any one of SEQ ID NOs. 13-27.

8. The use according to claim 6 or 7, wherein the primer and/or the probe capable of specifically recognizing and/or binding to the DNA region or the fragment thereof where the VIM gene is located comprise the nucleic acid sequences selected from any one of the following groups:
(1) primers: SEQ ID NOs. 13-14, probe: SEQ ID NO. 15;
(2) primers: SEQ ID NOs. 16-17, probe: SEQ ID NO. 18;
(3) primers: SEQ ID NOs. 19-20, probe: SEQ ID NO. 21;
(4) primers: SEQ ID NOs. 22-23, probe: SEQ ID NO. 24; and
(5) primers: SEQ ID NOs. 25-26, probe: SEQ ID NO. 27.

9. The use according to any one of claims 1-8, wherein the mutation(s) in the TERT gene comprise one or both selected from C250T and C228T.

10. The use according to claim 9, wherein the reagent combination comprises a primer and/or a probe capable of specifically recognizing and/or binding to the mutation C250T in the TERT gene.

11. The use according to claim 10, wherein the primer and/or the probe capable of specifically recognizing and/or binding to the mutation C250T in the TERT gene comprise the nucleic acid sequence as set forth in any one of SEQ ID NOs. 29-31.

12. The use according to any one of claims 1-11, wherein the reagent combination comprises a primer and/or a probe capable of specifically recognizing and/or binding to the mutation C228T in the TERT gene.

13. The use according to claim 12, wherein the primer and/or the probe capable of specifically recognizing and/or binding to the mutation C228T in the TERT gene comprise the nucleic acid sequence as set forth in any one of SEQ ID NOs. 28, 30, and 31.

14. The use according to any one of claims 1-13, wherein the mutation(s) in the FGFR3 gene comprise one or both selected from R248C and S249C.

15. The use according to claim 14, wherein the reagent combination comprises a primer and/or a probe capable of specifically recognizing and/or binding to the mutation R248C in the FGFR3 gene.

16. The use according to claim 15, wherein the primer and/or the probe capable of specifically recognizing and/or binding to the mutation R248C in the FGFR3 gene comprise the nucleic acid sequence as set forth in any one of SEQ ID NOs. 32, 34, and 35.

17. The use according to any one of claims 1-16, wherein the reagent combination comprises primers and/or a probe capable of specifically recognizing and/or binding to the mutation S249C in the FGFR3 gene.

18. The use according to claim 17, wherein the primer and/or probe capable of specifically recognizing and/or binding to the mutation S249C in the FGFR3 gene comprise the nucleic acid sequence as set forth in any one of SEQ ID NOs. 33-35.

19. The use according to any one of claims 1-18, wherein the reagent combination further comprises detecting the methylation level of a DNA region or a fragment thereof where a ONECUT2 gene is located in a sample to be tested.

20. The use according to claim 19, wherein the DNA region where the ONECUT2 gene is located comprises any one of the following groups: chr18:55107703-55107928, chr18:55108490-55108772, chr18:55108694-55108926, and/or chr18:55108912-55109114.

21. The use according to claim 19 or 20, wherein the reagent capable of detecting the methylation level of the DNA region or the fragment thereof where the ONECUT2 gene is located in the sample to be tested comprise a primer and/or a probe capable of specifically recognizing and/or binding to the DNA region or the fragment thereof where the ONECUT2 gene is located.

22. The use according to claim 21, wherein the primer and/or the probe capable of specifically recognizing and/or binding to the DNA region or the fragment thereof where the ONECUT2 gene is located comprise the nucleic acid sequence as set forth in any one of SEQ ID NOs. 1-12.

23. The use according to any one of claims 19-22, wherein the primer and/or the probe capable of specifically recognizing and/or binding to the DNA region or the fragment thereof where the ONECUT2 gene is located comprise the nucleic acid sequences selected from any one of the following groups:
(1) primers: SEQ ID NOs. 1-2, probe: SEQ ID NO. 3;
(2) primers: SEQ ID NOs. 4-5, probe: SEQ ID NO. 6;
(3) primers: SEQ ID NOs. 7-8, probe: SEQ ID NO. 9; and
(4) primers: SEQ ID NOs. 10-11, probe: SEQ ID NO. 12.

24. The use according to any one of claims 1-23, further comprising a reagent capable of specifically binding to a DNA region or a fragment thereof where a reference gene is located.

25. The use according to claim 24, wherein the reference gene is an ACTB gene.

26. The use according to claim 24 or 25, wherein the reagent capable of specifically binding to the DNA region or the fragment thereof where the reference gene is located comprise a primer and/or a probe, and the primer and/or the probe comprise the nucleic acid sequence as set forth in any one of SEQ ID NOs. 39-44.

27. The use according to any one of claims 1-26, further comprising a deamination reagent and/or a methylation-sensitive restriction endonuclease.

28. The use according to claim 27, wherein the deamination reagent includes bisulfite.
